(19) Europäisches Patentamt
European Patent Office
Office européen des brevets

(11) EP 1 398 319 B1

(12) # EUROPÄISCHE PATENTSCHRIFT

(45) Veröffentlichungstag und Bekanntmachung des Hinweises auf die Patenterteilung:
**09.11.2005 Patentblatt 2005/45**

(51) Int Cl.[7]: **C07F 9/655**

(21) Anmeldenummer: **03019803.0**

(22) Anmeldetag: **30.08.2003**

(54) **Chirale Monophosphorverbindungen und deren Übergangsmetallkomplexe**

Chiral monophosphorus compounds and transition metal complexes thereof

Composés monophosphore chiraux et leurs complexes de metaux transitoires

(84) Benannte Vertragsstaaten:
**AT BE BG CH CY CZ DE DK EE ES FI FR GB GR HU IE IT LI LU MC NL PT RO SE SI SK TR**

(30) Priorität: **12.09.2002 DE 10242351**

(43) Veröffentlichungstag der Anmeldung:
**17.03.2004 Patentblatt 2004/12**

(73) Patentinhaber: **LANXESS Deutschland GmbH
51369 Leverkusen (DE)**

(72) Erfinder:
- **Meseguer, Benjamin, Dr.
43007 Tarragona (ES)**
- **Militzer, Hans-Christian, Dr.
51519 Odenthal (DE)**
- **Castillon, Sergio, Prof. Dr.
43893 Altafulla / Tarragona (ES)**
- **Claver, Carmen, Prof. Dr.
43893 Altafulla / Tarragona (ES)**
- **Guiu, Ester
43001 Tarragona (ES)**

(56) Entgegenhaltungen:
**EP-A- 0 885 897** **WO-A-93/12260**
**WO-A-95/18787** **WO-A-96/16971**

- **RAJANBABU, T. V. ET AL: "Carbohydrate Phosphinites as Practical Ligands in Asymmetric Catalysis: Electronic Effects and Dependence of Backbone Chirality in Rh-Catalyzed Asymmetric Hydrogenations. Synthesis of R- or S-Amino Acids Using Natural Sugars as Ligand Precursors" JOURNAL OF ORGANIC CHEMISTRY (1997), 62(17), 6012-6028 , XP002216106**
- **RAJANBABU, T. V. ET AL: "Role of Electronic Asymmetry in the Design of New Ligands: The Asymmetric Hydrocyanation Reaction" JOURNAL OF THE AMERICAN CHEMICAL SOCIETY (1996), 118(26), 6325-6326 , XP002262100**
- **CHEMICAL ABSTRACTS, vol. 72, no. 17, 27. April 1970 (1970-04-27) Columbus, Ohio, US; abstract no. 90791, HOLY, ANTONIN: "Nucleic acid components and their analogs. CXXX. Preparation of nucleotide derivatives of 1'-homouridine and their behavior towards some nucleolytic enzymes" XP002262101 & COLLECTION OF CZECHOSLOVAK CHEMICAL COMMUNICATIONS (1970), 35(1), 81-8 , 1970,**
- **CLAVER C ET AL: "Biarylphosphonites: a class of monodentate phosphorus(III) ligands that outperform their chelating analogues in asymmetric hydrogenation catalysts" CHEMICAL COMMUNICATIONS - CHEMCOM, ROYAL SOCIETY OF CHEMISTRY, GB, Nr. 11, 7. Juni 2000 (2000-06-07), Seiten 961-962, XP002249649 ISSN: 1359-7345**
- **MANTELL S J ET AL: "3-HYDROXYMUSCARINES FROM L-RHAMNOSE" TETRAHEDRON, ELSEVIER SCIENCE PUBLISHERS, AMSTERDAM, NL, Bd. 49, Nr. 16, 16. April 1993 (1993-04-16), Seiten 3343-3358, XP002016535 ISSN: 0040-4020**
- **TAKAHASHI, S. ET AL.: "Total Synthesis of an Antitumor Agent , Muccin, Based on the "Chiron Approach"" J. ORG. CHEM., Bd. 67, Nr. 16, 7. September 2002 (2002-09-07), Seiten 5739-5752, XP002262276**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- DATABASE CROSSFIRE BEILSTEIN [Online] Beilstein Institut zur Förderung der Chemischen Wissenschaften, Frankfurt am Main, DE; Database accession no. BRN 44924 XP002262277 & BRIGL, GRUENER: CHEM. BER., Bd. 67, 1934, Seiten 1582-1586,
- DATABASE CROSSFIRE BEILSTEIN [Online] Beilstein Institut zur Förderung der Chemischen Wissenschaften, Frankfurt am Main, DE; Database accession no. BRN 1438485 XP002262278 & KOERNER ET AL.: J. BIOL. CHEM., Bd. 249, 1974, Seiten 5749-5751,
- DATABASE CROSSFIRE BEILSTEIN [Online] Beilstein Institut zur Förderung der Chemischen Wissenschaften, Frankfurt am Main, DE; Database accession no. BRN 5239417 XP002262279 & KOELL. P. ET AL: LIEBIGS ANN. CHEM. , 1987, Seiten 205-214,
- DATABASE CROSSFIRE BEILSTEIN [Online] Beilstein Institut zur Förderung der Chemischen Wissenschaften, Frankfurt am Main, DE; Database accession no. BRN 5239418 XP002262280 & GUTHRIE, R.D. ET AL.: AUST. J. CHEM., Bd. 35, Nr. 10, 1982, Seiten 2169-2173,
- DATABASE CROSSFIRE BEILSTEIN [Online] Beilstein Institut zur Förderung der Chemischen Wissenschaften, Frankfurt am Main, DE; Database accession no. BRN 1647462 XP002262281 & HASLEGRAVE ET AL.: TETRAHEDRON LETT., 1979, Seiten 2279-2280,
- DATABASE CROSSFIRE BEILSTEIN [Online] Beilstein Institut zur Förderung der Chemischen Wissenschaften, Frankfurt am Main, DE; Database accession no. BRN 5602645 XP002262282 & KUSZMANN, J. ET AL.: CARBOHYDR. RES, Bd. 123, 1983, Seiten 209-230,
- DATABASE CROSSFIRE BEILSTEIN [Online] Beilstein Institut zur Förderung der Chemischen Wissenschaften, Frankfurt am Main, DE; Database accession no. BRN 5767036; BRN 5767037 XP002262283 & OTERO, DARIO ET AL.: CARBOHYDR. RES, Bd. 128, 1984, Seiten 79-86,
- DATABASE CROSSFIRE BEILSTEIN [Online] Beilstein Institut zur Förderung der Chemischen Wissenschaften, Frankfurt am Main, DE; Database accession no. BRN 1688621 XP002262284 & TAKAMOTO, T. ET AL.: CARBOHYDR. RES, Bd. 60, 1978, Seiten 97-103,

## Beschreibung

Die vorliegende Erfindung betrifft chirale Monophosphorverbindungen und deren Übergangsmetallkomplexe, ein Verfahren zur Herstellung chiraler Monophosphorverbindungen und deren Übergangsmetallkomplexe sowie deren Verwendung in asymmetrischen Synthesen.

Enantiomerenangereicherte chirale Verbindungen sind wertvolle Ausgangssubstanzen zur Herstellung von Agrochemikalien und Pharmazeutika. Dabei hat die asymmetrische Katalyse für die Synthese solcher enantiomerenangereicherten chiralen Verbindungen eine große technische Bedeutung gewonnen.

Neuere Publikationen auf dem Gebiet der asymmetrischen Synthese zeigen deutlich, dass Übergangsmetallkomplexe von Monophosphorverbindungen als Katalysatoren in asymmetrisch geführten Reaktionen wie insbesondere in asymmetrischen Hydrierungen von C=O, C=N und C=C-Bindungen gut geeignet sind.

So ist beispielsweise aus A. Alexakis, Tetrahedron Asymmetry, 1997, 8, 3193-3196; W. Chen, J. Xiao, Tetrahedron Letters, 42, 2001, 2897-2899; M. Reetz, G. Mehler, Angew. Chem., 2000, 112, 4047-4049 und WO-A 01/94278 bekannt, optisch aktive Monophosphite bzw. deren Übergangsmetall-Komplexe für asymmetrische Hydrierungen einzusetzen.

Der Einsatz von optisch aktiven Monophosphoramiditen bzw. deren Übergangsmetall-Komplexe in asymmetrischen Synthesen ist beispielsweise aus M. van den Berg et al., J. Am. Chem. Soc., 2000, 122, 11539-11540, WO-A 02/04466; H. Waldmann, Chem. Eur. J. 2000, 6, 671-675; der Einsatz von chiralen Monophosphoniten beispielsweise aus C. Claver et al., Chem. Commun., 2000, 961-962 bekannt.

Nachteilig an den zitierten Monophosphorverbindungen ist, dass eine sterische und elektronische Variation des Ligandengerüstes, die für eine gezielte Optimierung und Adaptierung der Liganden und damit des Katalysators für ein vorgegebenes Substrat notwendig ist, nur in sehr begrenztem Umfang und nur durch zahlreiche, aufwändige Syntheseschritte möglich ist. Diese Nachteile schränken eine industrielle Nutzung solcher Liganden und der daraus herstellbaren Katalysatoren deutlich ein.

Es bestand daher das Bedürfnis, ein in seinen sterischen und elektronischen Eigenschaften leicht variierbares Ligandensystem basierend auf Monophosphorverbindungen zu entwickeln, dessen Übergangsmetallkomplexe als Katalysatoren in der asymmetrischen Synthese wie insbesondere asymmetrischen Hydrogenierungen hohe Enantioselektivitäten ermöglichen.

Es wurden nun Verbindungen der Formel (I) gefunden,

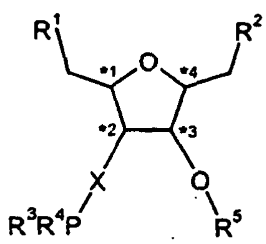

(I)

in der

- *1, *2, *3 und *4 jeweils unabhängig voneinander ein stereogenes Kohlenstoffatom markieren, das in R- oder S-Konfiguration vorliegt,
- X fehlt oder für Sauerstoff steht und
- $R^1$ und $R^2$ jeweils unabhängig voneinander stehen können für: Wasserstoff, $C_1$-$C_{20}$-Alkyl, $C_1$-$C_{20}$-Fluoralkyl, $C_2$-$C_{20}$-Alkenyl, $C_4$-$C_{24}$-Aryl, $C_5$-$C_{25}$-Arylalkyl, $C_6$-$C_{26}$-Arylalkenyl oder $NR^6R^7$, $OR^7$, -($C_1$-$C_8$-Alkyl)-$OR^7$, -($C_1$-$C_8$-Alkyl)-$NR^6R^7$ oder -$O_2CR^7$,

  wobei $R^6$ und $R^7$ jeweils unabhängig voneinander für $C_1$-$C_8$-Alkyl, $C_5$-$C_{15}$-Arylalkyl oder $C_4$-$C_{14}$-Aryl stehen oder $R^6$ und $R^7$ zusammen für einen cyclischen Aminorest mit insgesamt 4 bis 20 Kohlenstoffatomen steht,

  oder $R^1$ und $R^2$ jeweils unabhängig voneinander für Reste der Formel (IIa) stehen

$$-R^8\text{-}SiR^9R^{10}R^{11} \qquad \text{(IIa)}$$

in der

$R^8$ fehlt, für Sauerstoff oder Methylen steht und

$R^9$, $R^{10}$ und $R^{11}$ jeweils unabhängig voneinander für $C_1$-$C_{12}$-Alkyl, $C_5$-$C_{15}$-Arylalkyl oder $C_4$-$C_{14}$-Aryl stehen und

- $R^3$ und $R^4$ jeweils unabhängig voneinander für $R^{12}$, $OR^{13}$ oder $NR^{14}R^{15}$ stehen, wobei $R^{12}$, $R^{13}$, $R^{14}$ und $R^{15}$ jeweils unabhängig für $C_1$-$C_{12}$-Alkyl, $C_5$-$C_{15}$-Arylalkyl oder $C_4$-$C_{14}$-Aryl stehen oder $NR^{14}R^{15}$ zusammen für einen cyclischen Aminorest mit 4 bis 20 Kohlenstoffatomen steht oder $R^3$ und $R^4$ zusammen für -O-$R^{16}$-O- stehen, wobei $R^{16}$ für Reste steht, die ausgewählt sind aus der Gruppe $C_2$-$C_4$-Alkylen, 1,2-Phenylen, 1,3-Phenylen, 1,2-Cyclohexylen, 1,1'-Ferrocenylen, 1,2-Ferrocenylen, 2,2'-(1,1'-Binaphtylen), 2,2'-(1,1')-Biphenylen und 1,1'-(Diphenyl-2,2'-methylen)-diyl, wobei die genannten Reste gegebenenfalls einfach oder mehrfach durch Reste substituiert sein können, die ausgewählt sind aus der Gruppe Fluor, Chlor, $C_1$-$C_8$-Alkoxy und $C_1$-$C_8$-Alkyl und

- $R^5$ für Wasserstoff, $C_1$-$C_{20}$-Alkyl, $C_4$-$C_{24}$-Aryl, $C_5$-$C_{25}$-Arylalkyl, $C_1$-$C_{20}$-Halogenalkyl oder für Reste der Formel (IIb) steht

A-B-D (IIb)

in der

A fehlt oder für $C_1$-$C_{12}$-Alkylen steht

B für Funktionalitäten steht, die ausgewählt sind aus der Gruppe

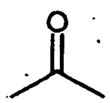 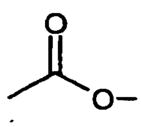 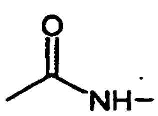 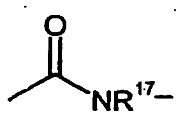

wobei

$R^{17}$ für $C_1$-$C_{20}$-Alkyl, $C_4$-$C_{24}$-Aryl, $C_5$-$C_{25}$-Arylalkyl steht

und

D für $C_1$-$C_8$-Alkyl, $C_4$-$C_{24}$-Aryl oder $C_5$-$C_{25}$-Arylalkyl steht oder

B und D für den Fall, dass A nicht fehlt zusammen für Cyano oder $[(C_1$-$C_8$-Alkylen)-O$]_n$-($C_1$-$C_8$-Alkyl) stehen können, wobei n eine ganze Zahl zwischen 1 und 8 ist oder

$R^{17}$ und D zusammen für einen cyclischen Aminorest mit 4 bis 12 Kohlenstoffatomen stehen.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

**Alkyl** beziehungsweise Alkylen beziehungsweise Alkoxy beziehungsweise Alkenyl bedeutet jeweils unabhängig einen geradkettigen, cyclischen, verzweigten oder unverzweigten Alkyl- beziehungsweise Alkylen- beziehungsweise Alkoxybeziehungsweise Alkenyl-Rest, wobei die genannten Reste gegebenenfalls weiterhin durch $C_1$-$C_4$-Alkoxyreste substituiert sein können.

Gleiches gilt für den nichtaromatischen Teil eines Arylalkyl-Restes.

$C_1$-$C_4$-Alkyl steht beispielsweise für Methyl, Ethyl, 2-Methoxyethyl, n-Propyl, iso-Propyl, n-Butyl, sec.-Butyl und tert.-Butyl, $C_1$-$C_8$-Alkyl darüber hinaus beispielsweise für n-Pentyl, 1-Methylbutyl, 2-Methylbutyl, 3-Methylbutyl, neoPentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, $C_1$-$C_{12}$-Alkyl weiter darüber hinaus beispielsweise für Adamantyl, die isomeren Menthyle, n-Nonyl, n-Decyl und n-

Dodecyl, $C_1$-$C_{20}$-Alkyl noch weiter darüber hinaus beispielsweise für n-Hexadecyl und n-Octadecyl.

$C_1$-$C_8$-Alkoxy steht beispielsweise für Methoxy, Ethoxy, 2-Methoxyethoxy, n-Propoxy, iso-Propoxy, n-Butoxy, sec.-Butoxy und tert.-Butoxy, n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy, $C_1$-$C_{12}$-Alkoxy weiter darüber hinaus beispielsweise für Adamantoxy, die isomeren Menthoxy-Reste, n-Decoxy und n-Dodecoxy.

$C_2$-$C_{20}$-Alkenyl steht beispielsweise für Vinyl, 1-Propenyl, iso-Propenyl, 1-Butenyl, 2-Butenyl, 1-Pentenyl, 2-Pentenyl, 2-Methyl-1-butenyl, 2-Methyl-2-butenyl, 3-Methyl-1-buteny, 1-Hexenyl, 1-Heptenyl, 1-Octenyl oder 2-Octenyl.

**Halogenalkyl** bedeutet jeweils unabhängig einen geradkettigen, cyclischen, verzweigten oder unverzweigten Alkyl-Rest, der einfach, mehrfach oder vollständig durch Chlor- oder Fluoratome substituiert ist.

Beispielsweise steht $C_1$-$C_{20}$-Halogenalkyl für Trifluormethyl, 2,2,2-Trichlorethyl, 2,2,2-Trifluorethyl, Pentafluorethyl, Nonafluorbutyl, Perfluoroctyl, Perfluordodecyl und Perfluorhexadecyl.

Aryl bedeutet jeweils unabhängig einen heteroaromatischen Rest mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 18 Gerüstkohlenstoffatomen.

Beispiele für carbocyclische aromatische Reste mit 6 bis 18 Gerüstkohlenstoffatomen sind Phenyl, Naphtyl, Phenanthrenyl, Anthracenyl oder Fluorenyl, heteroaromatische Reste mit 5 bis 18 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können sind beispielsweise Pyridinyl, Oxazolyl, Benzofuranyl, Dibenzofuran-yl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Chlor, Fluor, $C_1$-$C_{12}$-Alkyl, $C_1$-$C_{12}$-Halogenalkyl, $C_1$-$C_{12}$-Alkoxy, Di($C_1$-$C_8$-alkyl)amino, COO($C_1$-$C_8$-Alkyl), CON($C_1$-$C_8$-Alkyl)$_2$, COO($C_1$-$C_8$-Arylalkyl), COO($C_4$-$C_{14}$-Aryl), CO($C_1$-$C_8$-Alkyl), $C_5$-$C_{15}$-Arylalkyl oder Tri($C_1$-$C_6$-alkyl)siloxyl.

Gleiches gilt analog für **Aryloxy**-Reste.

**Arylalkyl** bedeutet jeweils unabhängig einen geradkettigen, cyclischen, verzweigten oder unverzweigten Alkyl-Rest, der einfach; mehrfach oder vollständig durch Aryl-Reste gemäß obiger Definition substituiert sein kann.

$C_5$-$C_{25}$-Arylalkyl steht beispielsweise für Benzyl, Diphenylbenzyl, Triphenylbenzyl (Trityl), 1-Phenylethyl, 1-Phenylpropyl, 2-Phenylpropyl, 1-Phenyl-1-methylethyl, 1-, 2-, 3- oder 4-Phenylbutyl, 1-Phenyl-1-methylpropyl, 1-Phenyl-2-methylpropyl, Phenyl-1,1-dimethylethyl, 1-,2-,3-,4- oder 5-Phenylpentyl, Phenyl-1-methylbutyl, Phenyl-2-methylbutyl, Phenyl-3-methylbutyl, Phenyl-2,2-dimethylpropyl, Phenyl-1-ethylpropyl, 1-Naphthylmethyl, 1-Naphthylethyl, Naphthyl-1-methylethyl, Naphthylbutyl, Naphthyl-1-methylpropyl, Naphthyl-2-methylpropyl, Naphthyl-1,1-dimethylethyl, Naphthylpentyl, Naphthyl-1-methylbutyl, Naphthyl-2-methylbutyl, Naphthyl-3-methylbutyl, Naphthyl-2,2-dimethylpropyl oder Naphthyl-1-ethylpropyl, sowie ihre isomeren oder stereoisomeren Formen.

**Arylalkenyl** bedeutet jeweils unabhängig einen geradkettigen, cyclischen, verzweigten oder unverzweigten Alkenyl-Rest, der einfach, mehrfach oder vollständig durch Aryl-Reste gemäß obiger Definition substituiert sein kann.

$C_6$-$C_{26}$-Arylalkenyl steht beispielsweise für 1-Phenylvinyl oder 2-Phenylvinyl.

Im Folgenden werden die bevorzugten Substitutionsmuster für Verbindungen der Formel (I) definiert:

$*^1$,$*^2$,$*^3$,$*^4$ definieren zusammen folgende Stereoisomere des zentralen substituierten Furanringes: (1R,2R,3R,4R), (1R,2R,3R,4S), (1R,2R,3S,4S), (1R,2S,3S,4S), (1R,2S,3R,4S), (1R,2S,3S,4R), (1R,2R,3S,4R), (1S,2S,3R,4S), (1S,2S,3S,4S), (1S,2S,3S,4R), (1S,2S,3R,4R), (1S,2R,3R,4R), (1S,2R,3S,4R), (1S,2R,3R,4S), (1S,2S,3R,4S), (1R,2R,3S,4R), bevorzugt (1R,2R,3R,4R), (1R,2R,3R,4S), (1R,2S,3S,4S), (1R,2S,3S,4R), (1R,2R,3S,4R), (1S,2S,3R,4S), (1S,2S,3S,4S), (1S,2S,3S,4R), (1S,2R,3R,4R), (1S,2R,3R,4S), (1S,2S,3R,4S), (1R,2R,3S,4R).

$R^1$ und $R^2$ stehen bevorzugt jeweils unabhängig voneinander für Wasserstoff, $C_1$-$C_4$-Alkyl, $C_4$-$C_{14}$-Aryl, O-$R^7$, $O_2$C-$R^7$, wobei $R^7$ vorzugsweise für $C_1$-$C_{12}$-Alkyl, $C_5$-$C_{25}$-Arylalkyl oder $C_4$-$C_{14}$-Aryl steht, oder OSi$R^9R^{10}R^{11}$, wobei $R^9$, $R^{10}$, und $R^{11}$ vorzugsweise jeweils unabhängig für $C_1$-$C_{12}$-Alkyl oder $C_4$-$C_{14}$-Aryl stehen.

$R^1$ und $R^2$ stehen besonders bevorzugt jeweils unabhängig voneinander für Wasserstoff, tert.-Butoxy, Trityloxy, tert-Butyldimethylsilyloxy, tert-Butyldiphenylsilyloxy, Trimethylsilyloxy, Triethylsilyloxy, Triisopropylsilyloxy, neo-Pentoxy oder 1-Adamantoxy.

Im Rahmen der Erfindung sind solche Verbindungen der Formel (I) jeweils bevorzugt, in denen $R^1$ und $R^2$

identisch sind.

$R^3$ und $R^4$ stehen bevorzugt jeweils unabhängig voneinander für $R^{12}$, $OR^{13}$ oder $NR^{14}R^{15}$, wobei $R^{12}$, $R^{13}$, $R^{14}$ und $R^{15}$ jeweils unabhängig für $C_1$-$C_{12}$-Alkyl oder $C_4$-$C_{14}$-Aryl stehen oder $NR^{14}R^{15}$ zusammen für einen cyclischen Aminorest mit 4 bis 12 Kohlenstoffatomen wie zum Beispiel Pyrrolidinyl oder Piperidinyl steht oder $R^3$ und $R^4$ zusammen für -O-$R^{16}$-O- stehen, wobei $R^{16}$ für Ethylen, 1,2-Phenylen, 1,3-Phenylen, 1,2-Cyclohexylen, 1,1'-Ferrocenylen, zweifach oder vierfach durch $C_1$-$C_8$-Alkyl substituiertes 1,1'-(Diphenyl-2,2'-methylen)-diyl, 1,2-Ferrocenylen, 2,2'-(1,1'-Binaphtylen) oder 2,2'-(1,1')-Biphenylen steht, wobei 2,2'-(1,1'-Binaphtylen) oder 2,2'-(1,1')-Biphenylen zumindest in 6,6'-Position durch Reste substituiert ist, die ausgewählt sind aus der Gruppe $C_1$-$C_8$-Alkoxy und $C_1$-$C_8$-Alkyl und weiterhin in 5,5'-,4,4'-, 3,3'- oder 2,2'-Position durch Reste substituiert sein kann, die ausgewählt sind aus der Gruppe Fluor, Chlor, $C_1$-$C_8$-Alkoxy und $C_1$-$C_8$-Alkyl.

$R^3$ und $R^4$ stehen besonders bevorzugt jeweils unabhängig voneinander für $R^{12}$, $OR^{13}$ oder $NR^{14}R^{15}$, wobei $R^{12}$ und $R^{13}$ jeweils unabhängig steht für Methyl, Ethyl, n-Propyl, iso-Propyl, tert.-Butyl, Cyclohexyl, Phenyl, 2-($C_1$-$C_8$)-alkylphenyl wie o-Tolyl, 3-($C_1$-$C_8$)-alkylphenyl wie m-Tolyl, 4-($C_1$-$C_8$)-alkylphenyl wie p-Tolyl, 2,6-Di-($C_1$-$C_8$)-alkylphenyl wie 2,6-Dimethylphenyl, 2,4-Di-($C_1$-$C_8$)-alkylphenyl wie 2,4-Dimethylphenyl, 3,5-Di-($C_1$-$C_8$)-alkylphenyl wie 3,5-Dimethylphenyl, 3,4,5-Tri-($C_1$-$C_8$)-alkylphenyl wie Mesityl und Isityl, 2-($C_1$-$C_8$)-Alkoxyphenyl wie o-Anisyl und o-Phenetyl, 3-($C_1$-$C_8$)-Alkoxyphenyl wie m-Anisyl und m-Phenetyl, 4-($C_1$-$C_8$)-Alkoxyphenyl wie p-Anisyl und p-Phenetyl, 2,4-Di-($C_1$-$C_8$)-alkoxyphenyl wie 2,4-Dimethoxyphenyl, 2,6-Di-($C_1$-$C_8$)-alkoxyphenyl wie 2,6-Dimethoxyphenyl, 3,5-Di-($C_1$-$C_8$)-Alkoxyphenyl wie 3,5-Dimethoxyphenyl, 3,4,5-Tri-($C_1$-$C_8$)-alkoxyphenyl wie 3,4,5-Trimethoxyphenyl, 3,5-Dialkyl-4-($C_1$-$C_8$)-alkoxyphenyl wie 3,5-Dimethyl-4-anisyl, 3,5-($C_1$-$C_8$)-Dialkyl-4-di-($C_1$-$C_8$)-alkylaminophenyl, 3,5-Dimethyl-4-dimethylamino-phenyl, 4-Di-($C_1$-$C_8$)-alkylaminophenyl wie 4-Diethylaminophenyl und 4-Dimethylaminophenyl, 3,5-Bis-[($C_1$-$C_4$)-fluoralkyl]phenyl wie 3,5-Bis-trifluormethylphenyl, 2,4-Bis-[($C_1$-$C_4$)-fluoralkyl]phenyl wie 2,4-Bis-trifluormethylphenyl, 4-[($C_1$-$C_4$)-Fluoralkyl]phenyl wie 4-Trifluormethylphenyl und ein-, zwei- drei-, vier- oder fünffach durch Fluor und/oder Chlor substituiertes Phenyl, Fluorenyl oder Naphthyl wie 4-Fluorphenyl und 4-Chlorphenyl und $NR^{14}R^{15}$ als Ganzes für Dimethylamino, Diethylamino, Pyrrolidino oder Diisopropylamino steht. Weiterhin stehen besonders bevorzugt $R^3$ und $R^4$ paarweise für O-$R^{16}$-O, wobei $R^{16}$ für 1,1'-Bis-(4,6-di-($C_1$-$C_8$-Alkyl)-phenyl)-2,2'-methylen)-diyl wie insbesondere 1,1'-Bis-(4-methyl-6-tert.-butyl-phenyl-2,2'-methylen)-diyl und 1,1'-Bis-(4-methyl-6-(1-methylcyclohexyl)-phenyl-2,2'-methylen)-diyl steht oder wobei $R^{16}$ für (R)-1,1'-Biphenyl-2,2'-diyl, (S)-1,1'-Biphenyl-2,2'-diyl, (R)-1,1'-Binaphthyl-2,2'-diyl, (S)-1,1'-Binaphthyl-2,2'-diyl, 1,1'-[Bis-(4-methyl-6-tert.-butyl-phenyl)-2,2'-methylen)]-diyl oder 1,1'-[Bis-(4-methyl-6-(1-methylcyclohexyl)-2,2'-methylen)]-diyl steht.

$R^3$ und $R^4$ stehen ganz besonders bevorzugt identisch für 2,4-Dimethylphenyl.

$R^5$ steht bevorzugt für Wasserstoff, $C_1$-$C_4$-Alkyl, -CO($C_1$-$C_4$-Alkyl), Benzyl-CO-Phenyl oder Phenyl, wobei Benzyl oder Phenyl gegebenenfalls einfach, zweifach oder dreifach durch Substituenten weiter substituiert sein kann, die ausgewählt sind aus der Gruppe $C_1$-$C_4$-Alkyl, $C_1$-$C_4$-Alkoxy oder $C_1$-$C_4$-Halogenalkyl.

$R^5$ steht besonders bevorzugt für Wasserstoff, Methyl oder Ethyl.

Besonders bevorzugte Verbindungen der Formel (I) sind solche der Formeln (Ia) bis (Id)

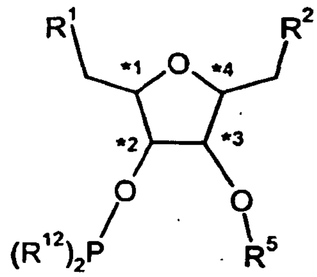

(Ia)

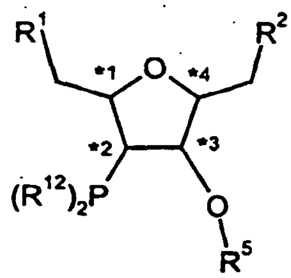

(Ib)

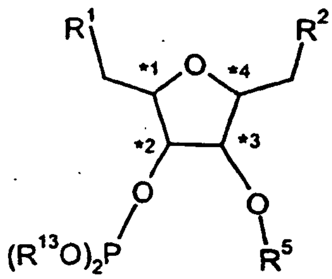

(Ic)

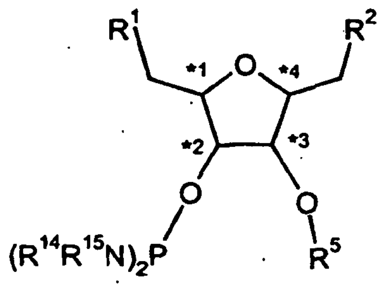

(Id)

in denen *1,*2,*3,*4, $R^1$, $R^2$, $R^5$, $R^{12}$, $R^{13}$, $R^{14}$ und $R^{15}$ die unter der Formel (I) genannte Bedeutung und Vorzugsbereiche besitzen.

Eine besonders bevorzugte Verbindung der Formel (I) ist 2-*O*-(Di(2,4-dimethylphenyl)phosphino)-1,6-di-*O*-(*tert*-butyldiphenylsilyl)-2,5-anhydro-D-mannit.

Der Begriff stereoisomerenangereichert umfasst im Sinne der Erfindung stereoisomerenreine Verbindungen oder auch Mischungen von stereoisomeren Verbindungen in denen ein Stereoisomeres in einem größeren relativen Anteil als das oder die anderen Stereoisomere, bevorzugt in einem relativen Anteil von 50 % bis 100 Mol-%, besonders bevorzugt 90 bis 100 Mol-% und ganz besonders bevorzugt 98 bis 100 Mol-%, vorliegt und umfasst insbesondere enantiomerenangereicherte Verbindungen für die die gleichen Definitionen gelten.

Die Verbindungen der Formel (I) beziehungsweise (Ia) bis (Id) können ausgehend von den bekannten 2,5-Anhydrocyclopentosen der Formel (III) hergestellt werden.

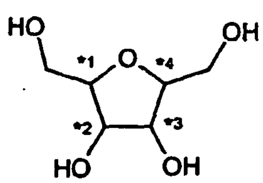

(III)

2,5-Anhydrocyclopentosen der Formel (III) sind beispielsweise: 2,5-Anhydro-D-mannit, 2,5-Anhydro-L-mannit, 2,5-Anhydro-L-iditol, 2,5-Anhydro-D-iditol, 2,5-Anhydro-L-glucitol, 2,5-Anhydro-D-glucitol, 2,5-Anhydro-altritol, 2,5-Anhydro-D-altritol, 2,5-Anhydro-galactitol, 2,5-Anhydro-allitol.

Als bevorzugte Verbindungen der allgemeinen Formel (III) seien genant: 2,5-Anhydro-D-mannit und 2,5-Anhydro-L-iditol. Im Rahmen der Erfindung sind insbesondere solche Verbindungen der Formel (I) bevorzugt, die ausgehend von 2,5-Anhydro-D-mannit und 2,5-Anhydro-L-iditol nach den nachfolgend beschriebenen Methoden erhältlich sind.

Die Verbindungen der Formel (III) können durch Umsetzung mit Verbindungen der Formel (IV)

$$R^{18}\text{-Hal} \quad \text{(IV)}$$

in der $R^{18}$ für $R^7$, $R^7CO$ oder $OSiR^9R^{10}R^{11}$ stehen und wobei $R^7$, $R^9$, $R^{10}$ und $R^{11}$ die unter der Formel (I) genannte Bedeutung und Vorzugsbereiche besitzen oder $R^{18}$ für $R^{19}$-$SO_2$- steht, wobei
$R^{19}$ für $C_1$-$C_{12}$-Alkyl, $C_1$-$C_{12}$-Fluoralkyl, $C_5$-$C_{25}$-Arylalkyl oder $C_4$-$C_{24}$-Aryl steht
und
Hal für Chlor, Brom oder Iod steht
in Verbindungen der Formel (V) überfuhrt werden,

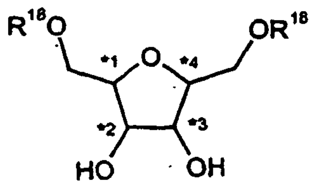

(V)

in der

$R^{18}$ jeweils unabhängig voneinander die unter der Formel (IV) genannte Bedeutung besitzt.

Verbindungen der Formel (V) in denen $R^{18}$ für $R^{19}SO_2$- steht, können weiterhin durch Umsetzung mit Aminen der Formel (VI)

$$HNR^6R^7 \quad \text{(VI)}$$

in der

$R^7$und $R^8$ jeweils unabhängig voneinander die unter der Formel (I) angegebenen Bedeutungen und Vorzugsbereiche besitzen in Verbindungen der Formel (VII) überführt werden,

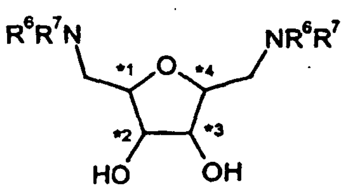

(VII)

in der

$R^6$ und $R^7$ jeweils unabhängig voneinander die unter der Formel (IV) genannte Bedeutung besitzen.

Weiterhin können Verbindungen der Formel (V) in denen $R^{18}$ für $R^{19}SO_2$- steht durch Umsetzung mit komplexen Hydriden der Formel (VIII),

$$Met^1(AlR^{20}_nR^{21}_{(4\text{-}n)}) \quad \text{(VIII)}$$

in der

$Met^1$ für Lithium, Natrium oder Kalium, bevorzugt für Lithium,

$R^{20}$ für Wasserstoff

n für 1, 2, 3 oder 4, bevorzugt für 4 und

$R^{21}$ für $C_1$-$C_4$-Alkyl steht

oder durch Umsetzung mit Organolithiumverbindungen der Formel (IX),

$$R^{20}\text{-Li} \tag{IX}$$

in der

$R^{20}$ für $C_1$-$C_{20}$-Alkyl, $C_1$-$C_{20}$-Fluoralkyl, $C_2$-$C_{20}$-Alkenyl, $C_4$-$C_{24}$-Aryl, $C_5$-$C_{25}$-Arylalkyl, $C_6$-$C_{26}$-Arylalkenyl, -($C_1$-$C_8$-Alkyl)-$OR^8$, -($C_1$-$C_8$-Alkyl)-$NR^7R^8$ oder (z.B. als cyclisches Acetal) geschütztes -($C_1$-$C_8$-Alkyl)-CO-$R^8$ steht zu Verbindungen der Formel (X) umgesetzt werden,

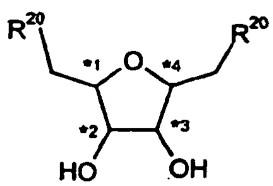

(X)

in der

$R^{20}$ die unter den Formeln (VIII) und (IX) angegebene Bedeutung besitzt.

Dabei wird aufgrund der Acidität der freien 2- und 3-Hydroxygruppen vorteilhafterweise ein Überschuss der Organolithiumverbindungen oder der komplexen Hydride eingesetzt oder die 3,4-Dioleinheit in an sich bekannter Weise durch Überführung beispielsweise in ein cyclisches Acetal geschützt und anschließend wieder entschützt.

Die Verbindungen der Formeln (V), (VII) und (X) umfassen zusammen die Verbindungen der Formel (XI), die als Zwischenprodukte zur Herstellung der erfindungsgemäßen Verbindungen der Formel (I) verwendet werden können.

In Formel (XI)

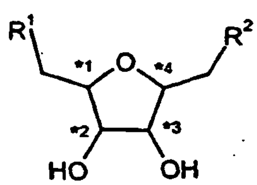

(XI)

besitzen $R^1$ und $R^2$ die gleiche Bedeutung und Vorzugsbereiche wie unter Formel (I) beschrieben.

Aus den Verbindungen der Formel (XI) können in prinzipiell bekannter Weise (siehe auch Rajan Babu, J. Org. Chem., 1997, 62, 6012-6028) durch Umsetzung mit Verbindungen der Formel (XII),

$$R^3R^4P\text{-Y} \tag{XII}$$

in der

$R^3$ und $R^4$ die gleiche Bedeutung und Vorzugsbereiche besitzen die unter der Formel (I) angegeben sind und
Y für Chlor, Brom, Iod, Dimethylamino oder Diethylamino, vorzugsweise für Chlor steht,

die Verbindungen der Formel (Ie) erhalten werden,

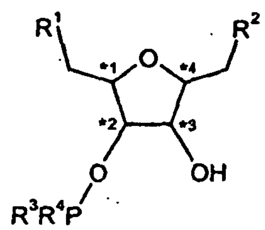

(Ie)

in der

$R^1$, $R^2$, $R^3$ und $R^4$ die gleiche Bedeutung und Vorzugsbereiche besitzen, wie sie unter der Formel (I) beschrieben sind.

Die Verbindungen der Formel (Ie) können weiterhin mit Verbindungen der Formel (XIII),

$$R^5Z \quad \text{(XIII)}$$

in der

$R^5$ die gleichen Bedeutungen und Vorzugsbereiche besitzen, die unter der Formel (I) angegeben sind und

Z für Chlor, Brom, Iod oder $R^{19}SO_3$ steht und im Falle dass $R^5$ über eine Carbonylgruppe gebunden sein soll, auch für $R^5O$- stehen kann

zu Verbindungen der Formel (If) umgesetzt werden,

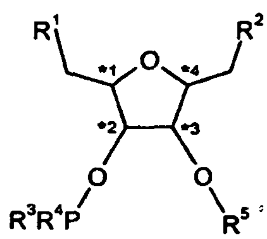

(If)

in denen

$R^1$, $R^2$, $R^3$, $R^4$, und $R^6$ die gleichen Bedeutungen und Vorzugsbereiche besitzen, die unter der Formel (I) angegeben sind und $R^5$ nicht für Wasserstoff steht.

Vorteilhafterweise erfolgt die Umsetzung zu Verbindungen der Formeln (Ie) bzw. (If) nach zumindest teilweiser Deprotonierung der Alkoholfunktion bzw. in Gegenwart einer Base die die Alkoholfunktion zumindest teilweise deprotonieren kann.

Bevorzugte Basen sind dabei für die Umsetzung zu Verbindungen der Formel (Ie) Amine oder N-Heteroaromaten wie insbesondere Pyridin, für die Umsetzung zu Verbindungen der Formel (If) Carbonate, Hydroxide, Alkoholate, Amide und Hydride von Alkali- oder Erdalkalimetallen, oder Amine oder N-Heteroaromaten wie insbesondere Pyridin.

Als Lösungsmittel für die Umsetzungen zu Verbindungen der Formel (Ie) eignen sich beispielsweise chlorierte Alkane wie Methylenchlorid, aliphatische Kohlenwasserstoffe wie z.B Hexan, Cyclohexan, gegebenenfalls chlorierte aromatische Kohlenwasserstoffe wie z.B. Chlorbenzol, Toluol, Pyridin, Benzol, Ketone wie z.B. Aceton oder Carbonsäureester wie z.B. Ethylacetat oder Dialkylether, wie z.B. THF oder Methyl-tert.-butylether. Bevorzugt wird als Lösungsmittel Methylenchlorid eingesetzt.

Als Lösungsmittel für die Umsetzung zu Verbindungen der Formel (If) eignen sich prinzipiell die gleichen Lösungsmittel wie für die Umsetzung zu Verbindungen der Formel (Ie), wobei bei Einsatz starker Basen wie Hydroxiden, Alkoholaten, Amiden und Hydriden vorteilhafterweise keine chlorierten Alkane verwendet werden.

Von der Erfindung sind insbesondere auch die Verbindungen der Formel (If) umfasst. Dabei gelten für die gleichen Bedeutungen und Vorzugsbereiche die unter der Formel (I) angegeben wurden.

In einer bevorzugten Ausführungsform werden die Verbindungen der Formel (If) jedoch hergestellt, indem man zunächst Verbindungen der Formel (Ie) mit Verbindungen der Formel (XIII) zu Verbindungen der Formel (XIV) umsetzt,

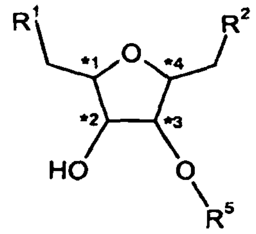

(XIV)

in der

$R^1$, $R^2$, $R^3$, $R^4$, und $R^6$ die gleichen Bedeutungen und Vorzugsbereiche besitzen, die unter der Formel (I) angegeben sind und $R^5$ nicht für Wasserstoff steht und die Verbindungen der Formel (XIV) dann mit Verbindungen der Formel (XII) zu Verbindungen der Formel (If) umsetzt.

Dabei gelten die gleichen Angaben für Lösungsmittel und Basen wie für das Verfahren über die Verbindungen der Formel (Ie).

Weiterhin können Verbindungen der Formel (Ib)

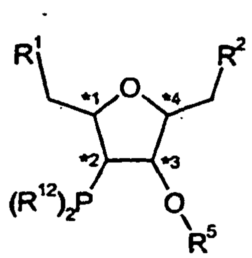

(Ib)

in der

$R^1$, $R^2$, $R^5$, $R^6$ und $R^{12}$ die unter der Formel (I) angegebenen Bedeutungen und Vorzugsbereiche besitzen durch ein erfindungsgemäßes Verfahren dadurch hergestellt werden, dass Verbindungen der Formel (XV)

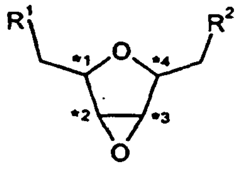

(XV)

in der

$R^1$ und $R^2$ die unter der Formel (I) genannte Bedeutung und Vorzugsbereiche besitzen in Gegenwart von Verbindungen der Formel (XVI),

$$(R^{12})_2PMet^2 \qquad (XVI)$$

in der

Met² für Lithium, Natrium oder Kalium steht und

$R^{12}$ die unter der Formel (I) genannte Bedeutung und Vorzugsbereiche besitzt

zu Verbindungen der Formel (XVII) umsetzt,

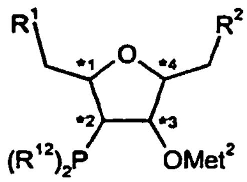

(XVII)

in der
$R^1$, $R^2$, $Met^2$ und $R^{12}$ die vorstehend genannte Bedeutung besitzen
und die Verbindungen der Formel (XVII) mit Verbindungen der Formel (XIII) mit der dort angegebenen Bedeutung zu Verbindungen der Formel (Ib) umsetzt.

Alternativ können die Verbindungen der Formel (XVII) durch Ansäuern in Verbindungen der Formel (Ib) überführt werden, in denen $R^5$ für Wasserstoff steht.

Weiterhin können die Verbindungen der Formel (Ib) beispielsweise dadurch hergestellt werden, dass die Verbindungen der Formel (XIV)

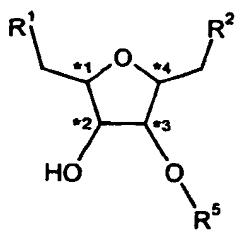

(XIV)

mit der vorstehend genannten Bedeutung zunächst in an sich bekannter Weise (siehe auch Terfort, Synthesis, 1992, 951-953) in Verbindungen der Formel (XVIII) überführt werden,

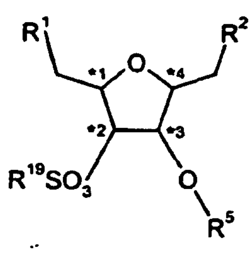

(XVIII)

in denen

$R^1$ und $R^2$ die unter der Formel (I) genannte Bedeutung und Vorzugsbereiche besitzt und $R^{19}$ die unter der Formel (IV) genannte Bedeutung und Vorzugsbereiche besitzt und die Verbindungen der Formel (XVIII) dann

mit Phosphiden der Formel (XVI) umsetzt.

Die Erfindung umfasst weiterhin Übergangsmetallkomplexe, die die erfindungsgemäßen Verbindungen der Formel (I) enthalten.

Übergangsmetallkomplexe sind bevorzugt solche von Ruthenium, Osmium, Cobalt, Rhodium, Iridium, Nickel, Palladium, Platin und Kupfer, bevorzugt solche von Ruthenium, Rhodium, Iridium, Nickel, Palladium, Platin und Kupfer.

Die erfindungsgemäßen Übergangsmetallkomplexe eignen sich insbesondere als Katalysatoren. Daher sind von der Erfindung auch Katalysatoren umfasst, die die erfindungsgemäßen Übergangsmetallkomplexe enthalten.

Als Katalysatoren können beispielsweise entweder isolierte Übergangsmetallkomplexe eingesetzt werden oder solche Übergangsmetallkomplexe, die durch Umsetzung von Übergangsmetallverbindungen und Verbindungen der Formel (I) erhältlich sind.

Isolierte Übergangsmetallkomplexe, die die Verbindungen der Formel (I) enthalten, sind vorzugsweise solche, in denen das Verhältnis von Übergangsmetall zu Verbindung der Formel (I) 1:2, 1:3 oder 1:4 beträgt.

Bevorzugt sind dabei die erfindungsgemäßen Verbindungen der Formel (XIX)

$$[(I)_4M] \qquad (XIX)$$

in der

(I) für Verbindungen der Formel (I) mit der dort genannten Bedeutung und deren Vorzugsbereichen steht und

M für Rhodium oder Iridium steht.

Bevorzugte Übergangsmetallkomplexe sind solche, die durch Umsetzung von Übergangsmetallverbindungen und Verbindungen der Formel (I) erhältlich sind.

Geeignete Übergangsmetallverbindungen sind beispielsweise solche der Formel (XXa)

$$M(An^1)_q \qquad (XXa)$$

in der

M für Rhodium, Iridium, Ruthenium, Nickel, Palladium, Platin oder Kupfer und

$An^1$ für Chlorid, Bromid, Acetat, Nitrat, Methansulfonat, Trifluormethansulfonat oder Acetylacetonat und

q für Rhodium, Iridium und Ruthenium für 3, für Nickel, Palladium und Platin für 2 und für Kupfer für 1 steht,

oder Übergangsmetallverbindungen der Formel (XXb)

$$M(An^2)_qL^1{}_2 \qquad (XXb)$$

in der

M für Ruthenium, Iridium, Ruthenium, Nickel, Palladium, Platin oder Kupfer und

$An^2$ für Chlorid, Bromid, Acetat, Methansulfonat oder Trifluormethansulfonat, Tetrafluoroborat oder Hexafluorophosphat, Perchlorat, Hexafluoroantimonat, Tetra(bis-3,5-trifluoromethylphenyl)-borat oder Tetraphenylborat steht und

q für Rhodium und Iridium für 1, für Ruthenium, Nickel, Palladium und Platin für 2 und für Kupfer für 1 steht,

$L^1$ jeweils für ein $C_2$-$C_{12}$-Alken wie beispielsweise Ethylen oder Cycloocten oder ein Nitril wie beispielsweise Acetonitril, Benzonitril oder Benzylnitril steht, oder

$L^1_2$ zusammen für ein ($C_4$-$C_{12}$)-Dien wie beispielsweise Bicyclo[2.1.1]hepta-2,5-dien (Norbomadien) oder 1,5-Cyclooctadien steht

oder Übergangsmetallverbindungen der Formel (XXc)

$$[ML^2An^1{}_2]_2 \qquad (XXc)$$

in der

M für Ruthenium und

$L^2$ für Arylreste wie zum Beispiel Cymol, Mesityl, Phenyl oder Cyclooctadien, Norbornadien oder Methylallyl steht

oder Übergangsmetallverbindungen der Formel (XXd)

$$Met^3{}_q[M(An^3)_4] \qquad (XXd)$$

in der

M für Palladium, Nickel, Iridium oder Rhodium und

$An^3$ für Chlorid oder Bromid steht und

$Met^3$ für Lithium, Natrium, Kalium, Ammonium oder organisches Ammonium steht und

q für Rhodium und Iridium für 3, für Nickel, Palladium und Platin für 2 steht,

oder Übergangsmetallverbindungen der Formel (XXe)

$$[M(L^3)_2]An^4 \qquad (XXe)$$

in der

M für Iridium oder Rhodium und

$L^3$ für ($C_4$-$C_{12}$)-Dien wie beispielsweise Bicyclo[2.1.1]hepta-2,5-dien (Norbornadien) oder 1,5-Cyclooctadien steht und

$An^4$ für ein nicht oder schwach koordinierendes Anion wie zum Beispiel Methansulfonat, Trifluormethansulfonat, Tetrafluoroborat, Hexafluorophosphat, Perchlorat, Hexafluoroantimonat, Tetra(bis-3,5-trifluromethylphenyl)-borat oder Tetraphenylborat steht.

Darüber hinaus sind als Übergangsmetallverbindungen beispielsweise Ni(1,5-Cyclooctadien)$_2$, $Pd_2$(dibenzylidenaceton)$_3$, Pd[$PPh_3$]$_4$, Cyclopentadienyl$_2$Ru, Rh(acac)(CO)$_2$, Ir(pyridin)2(1,5-Cyclooctadien), Cu(Phenyl)Br, Cu (Phenyl)Cl, Cu(Phenyl)I, Cu(PPh3)$_2$Br, [Cu($CH_3CN$)$_4$]$BF_4$ und [Cu($CH_3CN$)$_4$]$PF_6$ oder mehrkernige verbrückte Komplexe wie beispielsweise [Rh(1,5-cyclooctadien)Cl]$_2$, [Rh(1,5-cyclooctadien)Br]$_2$, [Rh(Ethen)$_2$Cl]$_2$, [Rh(Cyclooc-ten)$_2$Cl]$_2$ geeignet.

Bevorzugt werden als Übergangsmetallverbindungen eingesetzt:
[Rh(cod)Cl]$_2$, [Rh(cod)Br]$_2$, [Rh(cod)$_2$]$ClO_4$, [Rh(cod)$_2$]$BF_4$, [Rh(cod)$_2$]$PF_4$, [Rh(cod)$_2$]$ClO_6$, [Rh(cod)$_2$]OTf, [Rh(cod)$_2$] $BAr_4$ (Ar = 3,5-bistrifluormethylphenyl), [Rh(cod)$_2$]$SbF_6$, $RuCl_2$(cod), [(Cymol)$RuCl_2$]$_2$, [(Benzol)$RuCl_2$]$_2$, [(Mesityl) $RuCl_2$]$_2$, [(Cymol)$RuBr_2$]$_2$, [(Cymol)$RuI_2$]$_2$, [(Cymol)Ru($BF_4$)$_2$]$_2$, [(Cymol)Ru($PF_6$)$_2$]$_2$, [(Cymol)Ru($BAr_4$)$_2$]$_2$ (Ar = 3,5-bistrifluormethylphenyl), [(Cymol)Ru($SbF_6$)$_2$]$_2$, [Ir(cod)Cl]$_2$, [Ir(cod)$_2$]$PF_6$, [Ir(cod)$_2$]$ClO_4$, [Ir(cod)$_2$]$SbF_6$, [Ir(cod)$_2$]$BF_4$, [Ir (cod)$_2$]OTf, [Ir(cod)$_2$]$BAr_4$ (Ar = 3,5-bistrifluormethylphenyl), $RuCl_3$, $NiCl_3$, $RhCl_3$, $PdCl_2$, $PdBr_2$, Pd(OAc)2, $Pd_2$(dibenzylidenaceton)$_3$, Pd(acetylacetonat)$_2$, CuOTf, CuI, CuCl, Cu(OTf)$_2$, CuBr, CuI, $CuBr_2$, $CuCl_2$, $CuI_2$, [Rh(nbd)Cl]$_2$, [Rh

(nbd)Br]$_2$, [Rh(nbd)$_2$]$ClO_4$, [Rh(nbd)$_2$]$BF_4$, [Rh(nbd)$_2$]$PF_6$, [Rh(nbd)$_2$]OTf, [Rh(nbd)$_2$]$BAr_4$ (Ar = 3,5-bistrifluormethylphenyl), [Rh(nbd)$_2$]$SbF_6$, $RuCl_2$(nbd), [Ir(nbd)$_2$]$PF_6$, [Ir(nbd)$_2$]$ClO_4$, [Ir(nbd)$_2$]$SbF_6$, [Ir(nbd)$_2$]$BF_4$, [Ir(nbd)$_2$]OTf, [Ir(nbd)$_2$]$BAr_4$ (Ar = 3,5-bistrifluormethylphenyl), Ir(pyridin)$_2$(nbd), [Ru(DMSO)$_4$$Cl_2$], [Ru($CH_3CN$)$_4$$Cl_2$], [Ru(PhCN)$_4$$Cl_2$], [Ru(cod)$Cl_2$]$_n$, [Ru(cod)$_4$(Methallyl)$_2$], [Ru(acetylacetonat)$_3$]

Noch weiter bevorzugt sind [Rh(cod)Cl]$_2$, [Rh(cod)Br]$_2$, [Rh(cod)$_2$]$ClO_4$, [Rh(cod)$_2$]$BF_4$, [Rh(cod)$_2$]$PF_4$, [Rh(cod)$_2$]$ClO_6$, [Rh(cod)$_2$]OTf, [Rh(cod)$_2$]$BAr_4$ (Ar = 3,5-bistrifluormethylphenyl), [Rh(cod)$_2$]$SbF_6$, [Rh(nbd)Cl]$_2$, [Rh(nbd)Br]$_2$, [Rh(nbd)$_2$]$ClO_4$, [Rh(nbd)$_2$]$BF_4$, [Rh(nbd)$_2$]$PF_6$, [Rh(nbd)$_2$]OTf, [Rh(nbd)$_2$]$BAr_4$ (Ar = 3,5-bistrifluormethylphenyl), [Rh(nbd)$_2$]$SbF_6$, [Ir(cod)Cl]$_2$, [Ir(cod)$_2$]$PF_6$, [Ir(cod)$_2$]$ClO_4$, [Ir(cod)$_2$]$SbF_6$, [Ir(cod)$_2$]$BF_4$, [Ir(cod)$_2$]OTf, [Ir(cod)$_2$]$BAr_4$ (Ar = 3,5-bistrifluormethylphenyl).

Die Menge des Metalls in den eingesetzten Übergangsmetallverbindungen kann beispielsweise 5 bis 100 mol-% bezogen auf die eingesetzte Verbindung der Formel (I) betragen, bevorzugt sind 10 bis 50 mol-% und ganz besonders bevorzugt 15 bis 50 mol-%.

Die Katalysatoren, die die erfindungsgemäßen Übergangsmetallkomplexe enthalten eignen sich insbesondere für den Einsatz in einem Verfahren zur Herstellung von stereoisomerenangereicherten, bevorzugt enantiomerenangereicherten Verbindungen.

Bevorzugt werden die Katalysatoren für asymmetrische 1,4-Additionen, asymmetrische Hydroformylierungen, asymmetrische Hydrocyanierungen, asymmetrische Heck-Reaktionen und asymmetrische Hydrogenierungen eingesetzt, besonders bevorzugt für asymmetrische Hydrogenierungen.

Bevorzugte asymmetrische Hydrogenierungen sind beispielsweise Hydrogenierungen von prochiralen C=C-Bindungen wie zum Beispiel prochirale Enamine, Olefine, Enolether, C=O-Bindungen wie zum Beispiel prochirale Ketone und C=N-Bindungen wie zum Beispiel prochirale Imine. Besonders bevorzugte asymmetrische Hydrogenierungen sind Hydrogenierungen von prochiralen C=C-Bindungen wie zum Beispiel prochirale Enamine, Olefine, und C=N-Bindungen wie zum Beispiel prochirale Imine.

Von der Erfindung ist daher auch ein Verfahren zur Herstellung von stereoisomerenangereicherten, bevorzugt enantiomerenangereicherten Verbindungen durch katalytische Hydrierung von Olefinen, Enaminen, Enamiden, Iminen oder Ketonen umfasst, das dadurch gekennzeichnet ist, dass als Katalysatoren solche verwendet werden, die Übergangsmetallkomplexe von Verbindungen der Formel (I) mit der dort angegebenen Bedeutung verwendet werden.

Die Menge der eingesetzten Übergangsmetallverbindung oder des eingesetzten Übergangsmetallkomplexes kann beispielsweise 0.001 bis 5 mol-% bezogen auf das eingesetzte Substrat betragen, bevorzugt sind 0.001 bis 0,5 mol-%, ganz besonders bevorzugt 0.001 bis 0,1 mol-% und noch weiter bevorzugt 0,001 bis 0,008 mol-%.

In einer bevorzugten Ausführungsform können asymmetrische Hydrogenierungen beispielsweise so durchgeführt werden, dass der Katalysator aus einer Übergangsmetallverbindung und Verbindung der Formel (I) gegebenenfalls in einem geeigneten Lösungsmittel erzeugt wird, das Substrat zugegeben wird und die Reaktionsmischung bei Reaktionstemperatur unter Wasserstoffdruck gesetzt wird.

Besonders bevorzugt kommen für asymmetrische Hydrogenierungen als Metallverbindungen solche der allgemeinen Formel (XXI) zum Einsatz

$$[M(L^3)_2]An^4 \qquad (XXI)$$

wobei M für Rhodium oder Iridium steht und $L^3$ und An die oben genante Bedeutung besitzen,
oder zweikernige Komplexe wie zum Beispiel [Rh(1,5-cyclooctadien)Cl]$_2$, [Rh(1,5-cyclooctadien)Br]$_2$, [Rh(Ethen)$_2$Cl]$_2$, [Rh(Cycloocten)$_2$Cl]$_2$.

Besonders bevorzugte Metallverbindungen für asymmetrische Hydrogenierungen sind [Rh(cod)$_2$]OTf, [Rh(cod)$_2$]$BF_4$, [Rh(cod)$_2$]$PF_6$, [Rh(nbd)$_2$]$PF_6$, [Rh(nbd)$_2$]$BF_4$, und [Rh(Norbomadien)$_2$]OTf, [Ir(cod)$_2$]$BF_4$ und [Ir(cod)$_2$$PF_6$].

In einer besonders bevorzugten Ausführungsform werden in einem ausgeheizten Glasautoklaven werden Übergangsmetallverbindung und Verbindung der Formel (I) in entgastem Lösungsmittel gelöst. Man lässt ca. 5 min rühren und gibt anschließend das Substrat in entgastem Lösungsmittel zu. Nach dem Einstellen der jeweiligen Temperatur wird mit $H_2$ -Überdruck hydriert.

Als Lösungsmittel für die asymmetrische Katalyse eignen sich beispielsweise chlorierte Alkane wie Methylchlorid, kurzkettige $C_1$-$C_6$-Alkohole wie z.B. Methanol, iso-Propanol oder Ethanol, aromatische Kohlenwasserstoffe wie z.B. Toluol oder Benzol, Ketone wie z.B. Aceton oder Carbonsäureester wie z.B. Ethylacetat.

Die asymmetrische Katalyse wird vorteilhaft bei einer Temperatur von -20°C bis 200°C, bevorzugt 0 bis 100°C und besonders bevorzugt bei 20° bis 70°C durchgeführt.

Der Wasserstoffdruck kann beispielsweise 0,1 bis 200 bar, bevorzugt 0,5 bis 100 und besonders bevorzugt 1 bis 70 bar betragen.

Die erfindungsgemäßen Katalysatoren eignen sich insbesondere in einem Verfahren zur Herstellung von stereoisomerenangereicherten, bevorzugt enantiomerenangereicherten Wirkstoffen in Arzneimitteln und Agrochemikalien, oder Zwischenprodukten dieser beiden Klassen.

Der Vorteil der vorliegenden Erfindung ist, dass die Liganden in effizienter Weise hergestellt werden können und deren elektronische und sterische Eigenschaften ausgehend von einfach verfügbaren Edukten in weitem Masse variabel sind. Weiterhin zeigen die erfindungsgemäßen Liganden und deren Übergangsmetallkomplexe insbesondere in asymmetrischen Hydrogenierungen von C=C-Bindungen und Iminen hohe Enantioselektivitäten.

## Beispiele

### Beispiel 1:

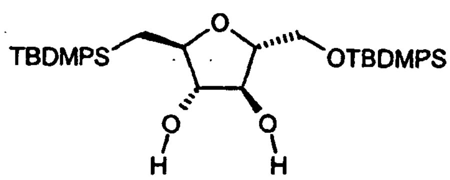

**1,6-Di-*O*-(*tert*-Butyldiphenylsilyl)-2,5-anhydro-D-mannit (B1):** 3 ml (11.66 mmol) *tert*-Butyldiphenylsilylchlorid (TBDMPSCl) wurde bei 0°C zu einer Lösung von 0.87 g (5.3 mmol) 2,5-Anhydro-D-Mannit und 1.5 g (22.28 mmol) Imidazol in 12 ml wasserfreiem DMF getropft. Die Mischung wurde auf Raumtemperatur erwärmt, 25 Stunden weiter gerührt und anschließend das Lösungsmittels im Vakuum entfernt. Die Mischung wurde mit $CH_2Cl_2$ verdünnt, mit Wasser gewaschen, die organische Phase über $Na_2SO_4$ getrocknet und das Lösungsmittel nachfolgend im Vakuum entfernt. Das Rohprodukt wurde mittels Säulenchromatographie gereinigt (Hexan / Essigester 4:1). Ausbeute 1.36 g (40% d.Th.). $^1$H NMR (400 MHz, $CDCl_3$) δ, 7.81-7.30 (m, 10H, Ph); 4.25 (m, 1H, H-3); 4.17 (m, 1H, H-2); 4.04 (d, 1H, OH); 3.86 (dd, 1H, $J_{6,2}$= 3.7 Hz, $J_{6,6'}$=11.1 Hz, H-6); 3.75 (dd, 1H, $J_{6',2}$= 3.2 Hz, $J_{6,6'}$=11.1 Hz, H-6'); 1.07 (s, 9H, C($\underline{H}_3$)$_3$);$^{13}$C NMR (100.6 MHz) δ, 136.10-126.99 (Ph), 87.09 (C-2), 79.71 (C-3), 65.52 (C-6), 26.73 (C($\underline{C}H_3$)$_3$), 19.02 ($\underline{C}$($CH_3$)$_3$).

### Beispiel 2:

**2-*O*-(Di(2,4-dimethylphenyl)phosphixio)-1,6-di-*O*-(*tert*-butyldiphenylsilyl)-2,5-anhydro-D-mannit (B2):**

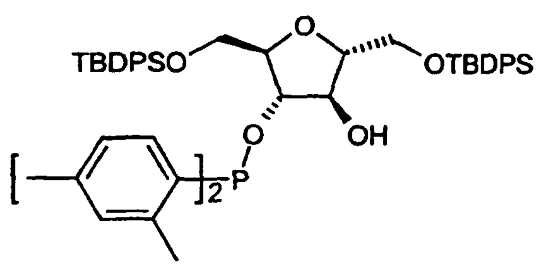

Zu einer Lösung von 300 mg (0.468 mmol) 1,6-di-*O*-(*tert*-butyldiphenylsilyl)-2,5-anhydro-D-mannit (**B1**) und 0.26 ml wasserfreiem $Et_3N$ (1.86 mmol) wurde eine Lösung von 169 mg (0.610 mmol) bis-(2,4-dimethylphenyl)-chlorphosphin in 2 ml wasserfreiem THF zugegeben und bei Raumtemperatur über Nacht gerührt. Nach Zugabe von Ethylether wurde die Mischung durch Celite® filtriert, das Lösungsmittel im Vakuum entfernt und das Rohprodukt wird mittels Säulenchromatographie gereinigt. Ausbeute 190 mg (49% d.Th.). $^1$H NMR (400 MHz, $CDCl_3$) δ, 7.59-6.87 (m, 26H, arom.), 4.47 (m, 1H, CH), 4.31 (m, 1H, CH), 3.99 (m, 2H, CH), 3.69 (m, 3H, $CH_2$), 3.54 (dd, 1H, $CH_2$), 2.79 (s, OH), 2.30 (s, 3H, $CH_3$), 2.17 (s, 3H, $CH_3$), 2.11 (s, 3H, $CH_3$), 2.06 (s, 3H, $CH_3$), 0.96 (s, 9H, $CH_3$), 0.94 (s, 9H, $CH_3$).

C NMR (75.4 MHz, $CDCl_3$) δ, 138.1-127.6 (CH, C, arom.), 86.0 ($^2J_{C-P}$=18 Hz, CH), 84.9 (CH), 83.9 ($^3J_{C-P}$=6.13 Hz, CH), 78.0 ($^2J_{C-P}$=4.5 Hz, CH CH), 64.7 ($CH_2$), 64.1 ($CH_2$), 27.1 ($CH_3$), 27.0 ($CH_3$), 21.4 (C), 20.5 (d, $^3J$=48.4 Hz, $CH_3$), 20.3 (d, $^3J$=48.4 Hz, $CH_3$), 19.6 (s, $CH_3$), 19.5 (s, $CH_3$). $^{31}P$ NMR (161.9 MHz, $CDCl_3$) δ, 102.9.

**Rhodium-katalysierte Hydrogenierung von Enamiden**

**Beispiele 3 und 4:**

0.01 oder 0.005 Moläquivalente Übergangsmetallverbindung und 0.027 Moläquivalente Ligand wurden unter Argon in entgastem $CH_2Cl_2$ gelöst (0.015 M) und bei Raumtemperatur 1/2 Stunde gerührt. Nach Zugabe von einem Moläquivalent Substrat in entgastem $CH_2Cl_2$ (0.08 M) unter Argon wurde die erhaltene Mischung in einem Autoklaven bei entsprechender Temperatur unter Wasserstoffdruck hydriert. Umsatz und ee wurden chromatographisch bestimmt.

**Ligand:**

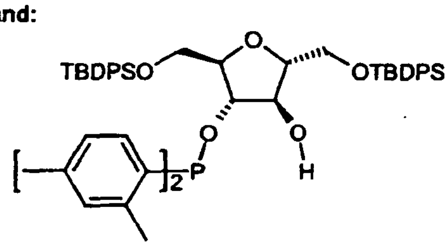

**Substrat:**

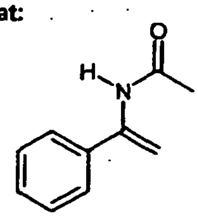

Die Ergebnisse der Hydrierungen sind in Tabelle 1 zusammengefasst.

Tabelle 1

| Beispiel | mol % Ligand | Metallprecursor | mol % Metallprecursor | T (°C) | P (bar) | Zeit (h) | Umsatz (%) | ee (%) |
|---|---|---|---|---|---|---|---|---|
| 3 | 2.7 | $[Rh(nbd)_2]PF_6$ | 1 | 25 | 3.5 | 16 | 96 | 18 |
| 4 | 2.7 | $[Rh(cod)Cl_2]_2$ | 0.5 | 25 | 30 | 24 | 100 | 83 |

**Patentansprüche**

**1.** Verbindungen der Formel (I),

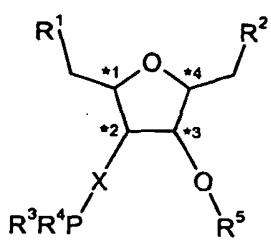

**(I)**

in der

- *1, *2, *3 und *4 jeweils unabhängig voneinander ein stereogenes Kohlenstoffatom markieren, das in R- oder S- Konfiguration vorliegt,
- X fehlt oder für Sauerstoff steht und
- $R^1$ und $R^2$ jeweils unabhängig voneinander stehen können für: Wasserstoff, $C_1$-$C_{20}$-Alkyl, $C_1$-$C_{20}$-Fluoralkyl, $C_2$-$C_{20}$-Alkenyl, $C_4$-$C_{24}$-Aryl, $C_5$-$C_{25}$-Arylalkyl, $C_6$-$C_{26}$-Arylalkenyl oder $NR^6R^7$, $OR^7$, -($C_1$-$C_8$-Alkyl)-$OR^7$,

$-(C_1-C_8\text{-Alkyl})-NR^6R^7$ oder $-O_2CR^7$,
wobei $R^6$ und $R^7$ jeweils unabhängig voneinander für $C_1$-$C_8$-Alkyl, $C_5$-$C_{15}$-Arylalkyl oder $C_4$-$C_{14}$-Aryl stehen oder $R^6$ und $R^7$ zusammen für einen cyclischen Aminorest mit insgesamt 4 bis 20 Kohlenstoffatomen steht, oder $R^1$ und $R^2$ jeweils unabhängig voneinander für Reste der Formel (IIa) stehen

$$-R^8-SiR^9R^{10}R^{11} \qquad \text{(IIa)}$$

in der

$R^8$ fehlt, für Sauerstoff oder Methylen steht und

$R^9$, $R^{10}$ und $R^{11}$ jeweils unabhängig voneinander für $C_1$-$C_{12}$-Alkyl, $C_5$-$C_{15}$-Arylalkyl oder $C_4$-$C_{14}$-Aryl stehen und

- $R^3$ und $R^4$ jeweils unabhängig voneinander für $R^{12}$, $OR^{13}$ oder $NR^{14}R^{15}$ stehen, wobei $R^{12}$, $R^{13}$, $R^{14}$ und $R^{15}$ jeweils unabhängig für $C_1$-$C_{12}$-Alkyl, $C_5$-$C_{15}$-Arylalkyl oder $C_4$-$C_{14}$-Aryl stehen oder $NR^{14}R^{15}$ zusammen für einen cyclischen Aminorest mit 4 bis 20 Kohlenstoffatomen steht oder $R^3$ und $R^4$ zusammen für $-O-R^{16}-O-$ stehen, wobei $R^{16}$ für Reste steht, die ausgewählt sind aus der Gruppe $C_2$-$C_4$-Alkylen, 1,2-Phenylen, 1,3-Phenylen, 1,2-Cyclohexylen, 1,1'-Ferrocenylen, 1,2-Ferrocenylen, 2,2'-(1,1'-Binaphtylen), 2,2'-(1,1')-Biphenylen und 1,1'-(Diphenyl-2,2'-methylen)-diyl, wobei die genannten Reste gegebenenfalls einfach oder mehrfach durch Reste substituiert sein können, die ausgewählt sind aus der Gruppe Fluor, Chlor, $C_1$-$C_8$-Alkoxy und $C_1$-$C_8$-Alkyl und

- $R^5$ für Wasserstoff, $C_1$-$C_{20}$-Alkyl, $C_4$-$C_{24}$-Aryl, $C_5$-$C_{25}$-Arylalkyl, $C_1$-$C_{20}$-Halogenalkyl oder für Reste der Formel (IIb) steht

$$A-B-D \qquad \text{(IIb)}$$

in der

A fehlt oder für $C_1$-$C_{12}$-Alkylen steht

B für Funktionalitäten steht, die ausgewählt sind aus der Gruppe

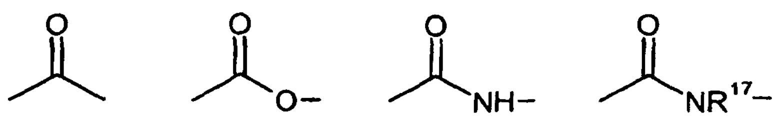

wobei

$R^{17}$ für $C_1$-$C_{20}$-Alkyl, $C_4$-$C_{24}$-Aryl, $C_5$-$C_{25}$-Arylalkyl stehen kann

und

D für $C_1$-$C_8$-Alkyl, $C_4$-$C_{24}$-Aryl oder $C_5$-$C_{25}$-Arylalkyl steht oder

B und D für den Fall, dass A nicht fehlt zusammen für Cyano oder $[(C_1\text{-}C_8\text{-Alkylen})\text{-}O]_n\text{-}(C_1\text{-}C_8\text{-Alkyl})$ stehen können, wobei n eine ganze Zahl zwischen 1 und 8 ist oder

$R^{17}$ und D zusammen für einen cyclischen Aminorest mit 4 bis 12 Kohlenstoffatomen stehen.

**2.** Verbindungen gemäß Anspruch 1, **dadurch gekennzeichnet, dass** *1, *2, *3 und *4 zusammen folgende Stereoisomere des zentralen substituierten Furanringes definieren:
(1R,2R,3R,4R), (1R,2R,3R,4S), (1R,2S,3S,4S), (1R,2S,3S,4R), (1R,2R,3S,4R), (1S,2S,3R,4S), (1S,2S,3S,4S), (1S,2S,3S,4R), (1S,2R,3R,4R), (1S,2R,3R,4S), (1S,2S,3R,4S), (1R,2R,3S,4R).

**3.** Verbindungen gemäß mindestens einem der Ansprüche 1 und 2, **dadurch gekennzeichnet, dass** $R^1$ und $R^2$ jeweils unabhängig voneinander für Wasserstoff, tert.-Butoxy, Trityloxy, tert-Butyldimethylsilyloxy, tert-Butyldiphenylsilyloxy, Trimethylsilyloxy, Triethylsilyloxy, Triisopropylsilyloxy, neo-Pentoxy oder 1-Adamantoxy stehen.

**4.** Verbindungen gemäß mindestens einem der Ansprüche 1 bis 3, **dadurch gekennzeichnet, dass** $R^1$ und $R^2$ identisch sind.

**5.** Verbindungen gemäß mindestens einem der Ansprüche 1 bis 4, **dadurch gekennzeichnet, dass** $R^3$ und $R^4$ jeweils unabhängig voneinander für $R^{12}$, $OR^{13}$ oder $NR^{14}R^{15}$ stehen , wobei $R^{12}$, $R^{13}$, $R^{14}$ und $R^{15}$ jeweils unabhängig für $C_1$-$C_{12}$-Alkyl oder $C_4$-$C_{14}$-Aryl stehen oder $NR^{14}R^{15}$ zusammen für einen cyclischen Aminorest mit 4 bis 12 Kohlenstoffatomen wie zum Beispiel Pyrrolidinyl oder Piperidinyl steht oder $R^3$ und $R^4$ zusammen für -O-$R^{16}$-O-stehen, wobei $R^{16}$ für Ethylen, 1,2-Phenylen, 1,3-Phenylen, 1,2-Cyclohexylen, 1,1'-Ferrocenylen, zweifach oder vierfach durch $C_1$-$C_8$-Alkyl substituiertes 1,1'-(Diphenyl-2,2'-methylen)-diyl, 1,2-Ferrocenylen, 2,2'-(1,1'-Binaphtylen) oder 2,2'-(1,1')-Biphenylen steht, wobei 2,2'-(1,1'-Binaphtylen) oder 2,2'-(1,1')-Biphenylen zumindest in 6,6'-Position durch Reste substituiert ist, die ausgewählt sind aus der Gruppe $C_1$-$C_8$-Alkoxy und $C_1$-$C_8$-Alkyl und weiterhin in 5,5'-,4,4'-, 3,3'- oder 2,2'-Position durch Reste substituiert sein kann, die ausgewählt sind aus der Gruppe Fluor, Chlor, $C_1$-$C_8$-Alkoxy und $C_1$-$C_8$-Alkyl.

**6.** Verbindungen gemäß mindestens einem der Ansprüche 1 bis 5, **dadurch gekennzeichnet, dass** $R^5$ für Wasserstoff, $C_1$-$C_4$-Alkyl, -CO($C_1$-$C_4$-Alkyl), Benzyl-COPhenyl oder Phenyl steht, wobei Benzyl oder Phenyl gegebenenfalls einfach, zweifach oder dreifach durch Substituenten weiter substituiert sein kann, die ausgewählt sind aus der Gruppe $C_1$-$C_4$-Alkyl, $C_1$-$C_4$-Alkoxy oder $C_1$-$C_4$-Halogenalkyl.

**7.** Verbindungen gemäß mindestens einem der Ansprüche 1 bis 6, **dadurch gekennzeichnet, dass** sie den Formeln (Ia) bis (Id) gehorchen

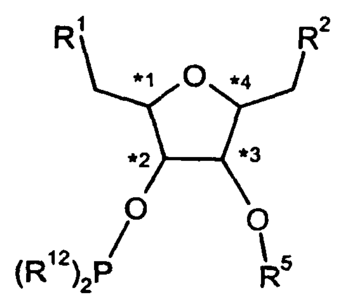

(Ia)

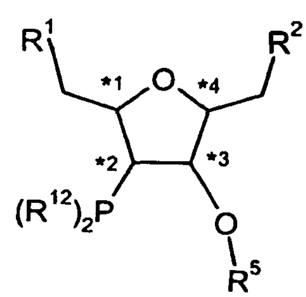

(Ib)

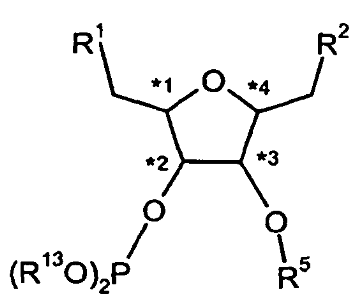

(Ic)

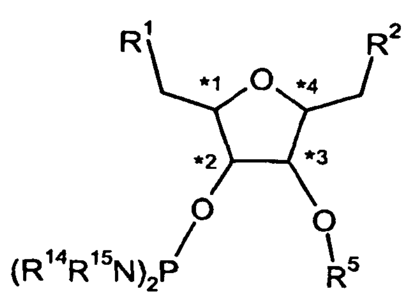

(Id)

in denen *1,*2,*3,*4, $R^1$, $R^2$, $R^5$, $R^{12}$, $R^{13}$, $R^{14}$ und $R^{15}$ die unter der Formel (I) genannte Bedeutung besitzen.

**8.** 2-*O*-(Di(2,4-dimethylphenyl)phosphino)-1,6-di-*O*-(*tert*-butyldiphenylsilyl)-2,5-anhydro-D-mannit.

**9.** Verfahren zur Herstellung von Verbindungen der Formel (Ib)

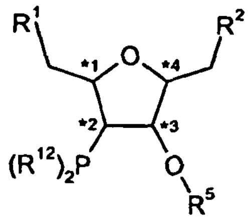

(Ib)

in der

$R^1$, $R^2$, $R^5$, $R^6$ und $R^{12}$ die unter der Formel (I) in Anspruch 1 angegebenen Bedeutungen besitzen, **dadurch gekennzeichnet, dass** Verbindungen der Formel (XV)

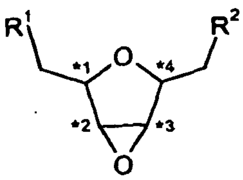

(XV)

in der

$R^1$ und $R^2$ die unter der Formel (I) genannte Bedeutung besitzen in Gegenwart von Verbindungen der Formel (XVI),

$$(R^{12})_2PMet^2 \quad (XVI)$$

in der

$Met^2$ für Lithium, Natrium oder Kalium steht und

$R^{12}$ die unter der Formel (I) genannte Bedeutung und Vorzugsbereiche besitzt

zu Verbindungen der Formel (XVII) umgesetzt werden,

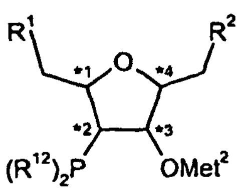

(XVII)

in der
$R^1$, $R^2$, $Met^2$ und $R^{12}$ die vorstehend genannte Bedeutung besitzen
und die Verbindungen der Formel (XVII) mit Verbindungen der Formel (XIII) umsetzt,

$$R^5Z \quad \text{(XIII)}$$

in der

$R^5$ die gleichen Bedeutungen und Vorzugsbereiche besitzen, die unter der Formel (I) angegeben sind und

Z für Chlor, Brom, Iod oder $R^{19}SO_3$ steht, wobei $R^{19}$ für $C_1$-$C_{12}$-Alkyl, $C_1$-$C_{12}$-Halogenalkyl, $C_5$-$C_{25}$-Arylalkyl oder $C_4$-$C_{24}$-Aryl steht und im Falle dass $R^5$ über eine Carbonylgruppe gebunden sein soll, auch für $R^5O$-stehen kann.

**10.** Übergangsmetallkomplexe enthaltend Verbindungen gemäß einem oder mehreren der Ansprüche 1 bis 8.

**11.** Übergangsmetallkomplexe gemäß Anspruch 10, **dadurch gekennzeichnet, dass** das Übergangsmetall ausgewählt ist aus der Gruppe Ruthenium, Osmium, Cobalt, Rhodium, Iridium, Nickel, Palladium, Platin und Kupfer.

**12.** Übergangsmetallkomplexe gemäß mindestens einem der Ansprüche 10 bis 11, **dadurch gekennzeichnet, dass** das molare Verhältnis von Übergangsmetall zu Verbindungen gemäß mindestens einem der Ansprüche 1 bis 8 1:2, 1:3 oder 1:4 beträgt.

**13.** Übergangsmetallkomplexe gemäß mindestens einem der Ansprüche 10 bis 11, **dadurch gekennzeichnet, dass** sie der Formel (XIX) gehorchen

$$[(I)_4M] \quad \text{(XIX)}$$

in der

(I) für Verbindungen der Formel (I) mit der in Anspruch 1 genannten Bedeutung und

M für Rhodium oder Iridium steht.

**14.** Übergangsmetallkomplexe gemäß mindestens einem der Ansprüche 10 bis 11, **dadurch gekennzeichnet, dass** sie durch Umsetzung von Übergangsmetallverbindungen und Verbindungen gemäß mindestens einem der Ansprüche 1 bis 8 gewonnen werden.

**15.** Übergangsmetallkomplexe gemäß Anspruch 14, **dadurch gekennzeichnet, dass** als Übergangsmetallverbindungen eingesetzt werden:

Übergangsmetallverbindungen der Formel (XXa)

$$M(An^1)_q \quad \text{(XXa)}$$

in der

M für Rhodium, Iridium, Ruthenium, Nickel, Palladium, Platin oder Kupfer und

$An^1$ für Chlorid, Bromid, Acetat, Nitrat, Methansulfonat, Trifluormethansulfonat oder Acetylacetonat und

q für Rhodium, Iridium und Ruthenium für 3, für Nickel, Palladium und Platin für 2 und für Kupfer für 1 steht,

oder Übergangsmetallverbindungen der Formel (XXb)

$$M(An^2)_qL^1{}_2 \quad \text{(XXb)}$$

in der

M für Ruthenium, Iridium, Ruthenium, Nickel, Palladium, Platin oder Kupfer und

$An^2$ für Chlorid, Bromid, Acetat, Methansulfonat oder Trifluormethansulfonat, Tetrafluoroborat oder Hexafluorophosphat, Perchlorat, Hexafluoroantimonat, Tetra(bis-3,5-trifluoromethylphenyl)-borat oder Tetraphenylborat steht und

q für Rhodium und Iridium für 1, für Ruthenium, Nickel, Palladium und Platin für 2 und für Kupfer für 1 steht,

$L^1$ jeweils für ein $C_2$-$C_{12}$-Alken wie beispielsweise Ethylen oder Cycloocten oder ein Nitril wie beispielsweise Acetonitril, Benzonitril oder Benzylnitril steht, oder

$L^1{}_2$ zusammen für ein ($C_4$-$C_{12}$)-Dien wie beispielsweise Bicyclo[2.1.1]hepta-2,5-dien (Norbomadien) oder 1,5-Cyclooctadien steht

oder Übergangsmetallverbindungen der Formel (XXc)

$$[ML^2An^1{}_2]_2 \qquad \text{(XXc)}$$

in der

M für Ruthenium und

$L^2$ für Arylreste wie zum Beispiel Cymol, Mesityl, Phenyl oder Cyclooctadien, Norbomadien oder Methylallyl steht

oder Übergangsmetallverbindungen der Formel (XXd)

$$Met^3{}_q[M(An^3)_4] \qquad \text{(XXd)}$$

in der

M für Palladium, Nickel, Iridium oder Rhodium und

$An^3$ für Chlorid oder Bromid steht und

$Met^3$ für Lithium, Natrium, Kalium, Ammonium oder organisches Ammonium steht und

q für Rhodium und Iridium für 3, für Nickel, Palladium und Platin für 2 steht,

oder Übergangsmetallverbindungen der Formel (XXe)

$$[M(L^3)_2]An^4 \qquad \text{(XXe)}$$

in der

M für Iridium oder Rhodium und

$L^3$ für ($C_4$-$C_{12}$)-Dien wie beispielsweise Bicyclo[2.1.1]hepta-2,5-dien (Norbomadien) oder 1,5-Cyclooctadien steht und

$An^4$ für ein nicht oder schwach koordinierendes Anion wie zum Beispiel Methansulfonat, Trifluormethansulfonat, Tetrafluoroborat, Hexafluorophosphat, Perchlorat, Hexafluoroantimonat, Tetra(bis-3,5-trifluoromethylphenyl)-borat oder Tetraphenylborat steht,

oder Ni(1,5-Cyclooctadien)$_2$, $Pd_2$(dibenzylidenaceton)$_3$, Pd[$PPh_3$]$_4$, Cyclopentadienyl$_2$Ru, Rh(acac)(CO)$_2$, Ir (pyridin)2(1,5-Cyclooctadien), Cu(Phenyl)Br, Cu(Phenyl)Cl, Cu(Phenyl)I, Cu($PPh_3$)$_2$Br, [Cu($CH_3CN$)$_4$]$BF_4$ und [Cu($CH_3CN$)$_4$]$PF_6$ oder mehrkernige verbrückte Komplexe wie beispielsweise [Rh(1,5-cyclooctadien)Cl]$_2$, [Rh(1,5-cyclooctadien)Br]$_2$, [Rh(Ethen)$_2$Cl]$_2$, und [Rh(Cycloocten)$_2$Cl]$_2$.

**16.** Übergangsmetallkomplexe gemäß mindestens einem der Ansprüche 14 bis 15, **dadurch gekennzeichnet, dass** die Menge des Metalls in den eingesetzten Übergangsmetallverbindungen 5 bis 100 mol-% bezogen auf die eingesetzte Verbindung gemäß mindestens einem der Ansprüche 1 bis 8 beträgt.

**17.** Katalysatoren enthaltend Übergangsmetallkomplexe gemäß mindestens einem der Ansprüche 10 bis 16.

**18.** Verwendung von Übergangsmetallkomplexen gemäß einem oder mehreren der Ansprüche 10 bis 16 oder Katalysatoren gemäß Anspruch 17 zur Herstellung von stereoisomerenangereicherten Verbindungen.

**19.** Verwendung gemäß Anspruch 18, **dadurch gekennzeichnet, dass** die stereoisomerenangereicherte Verbindungen durch asymmetrische 1,4-Additionen, asymmetrische Hydroformylierungen, asymmetrische Hydrocyanierungen, asymmetrische Heck-Reaktionen und asymmetrische Hydrogenierungen erhalten werden.

**20.** Verwendung gemäß einem oder mehreren der Ansprüche 18 und 19, **dadurch gekennzeichnet, dass** die stereoisomerenangereicherten Verbindungen zur Herstellung von Wirkstoffen in Arzneimitteln und Agrochemikalien, oder Zwischenprodukten dieser beiden Klassen verwendet werden.

**21.** Verfahren zur Herstellung von stereoisomerenangereicherten Verbindungen durch katalytische Hydrierungen von Olefinen, Enaminen, Enamiden, Iminen oder Ketonen, 1,4-Additionen, Hydroformylierungen, Hydrocyanierungen oder Heck-Reaktionen, **dadurch gekennzeichnet, dass** als Katalysatoren solche verwendet werden, die Übergangsmetallkomplexe gemäß einem oder mehreren der Ansprüche 10 bis 16 enthalten.

**22.** Verfahren gemäß Anspruch 21, **dadurch gekennzeichnet, dass** die Menge der eingesetzten Übergangsmetallkomplexe 0.001 bis 5 mol-% bezogen auf das eingesetzte Substrat beträgt.

**23.** Verfahren gemäß einem oder mehreren der Ansprüche 21 und 22, **dadurch gekennzeichnet, dass** die stereoisomerenangereicherten Verbindungen durch katalytische Hydrierung von Olefinen, Enamiden oder Iminen gewonnen werden.

**24.** Verfahren gemäß einem oder mehreren der Ansprüche 21 bis 23, **dadurch gekennzeichnet, dass** bei einer Temperatur von 20°C bis 200°C gearbeitet wird.

**25.** Verfahren gemäß einem oder mehreren der Ansprüche 21 bis 24, **dadurch gekennzeichnet, dass** der Wasserstoffdruck 0,1 bis 200 bar beträgt.

## Claims

**1.** Compounds of the formula (I)

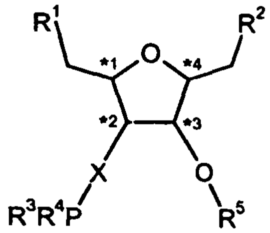

**(I)**

where

- *1, *2, *3 and *4 are each independently a stereogenic carbon atom which has R- or S- configuration,
- X is absent or is oxygen and
- $R^1$ and $R^2$ may each independently be hydrogen, $C_1$-$C_{20}$-alkyl, $C_1$-$C_{20}$-fluoroalkyl, $C_2$-$C_{20}$-alkenyl, $C_4$-$C_{24}$-aryl, $C_5$-$C_{25}$-arylalkyl, $C_6$-$C_{26}$-arylalkenyl or $NR^6R^7$, $OR^7$, -($C_1$-$C_8$-alkyl)-$OR^7$, -($C_1$-$C_8$-alkyl)-$NR^6R^7$ or $-O_2CR^7$,
  where $R^6$ and $R^7$ are each independently $C_1$-$C_8$-alkyl, $C_5$-$C_{15}$-arylalkyl or $C_4$-$C_{14}$-aryl, or $R^6$ and $R^7$ together are a cyclic amino radical having a total of 4 to 20 carbon atoms,
  or $R^1$ and $R^2$ are each independently radicals of the formula (IIa)

$$-R^8\text{-}SiR^9R^{10}R^{11} \qquad \text{(IIa)}$$

  where

  $R^8$ is absent or is oxygen or methylene and

  $R^9$, $R^{10}$ and $R^{11}$ are each independently $C_1$-$C_{12}$-alkyl, $C_5$-$C_{15}$-arylalkyl or $C_4$-$C_{14}$-aryl and

- $R^3$ and $R^4$ are each independently $R^{12}$, $OR^{13}$ or $NR^{14}R^{15}$ where $R^{12}$, $R^{13}$, $R^{14}$ and $R^{15}$ are each independently $C_1$-$C_{12}$-alkyl, $C_5$-$C_{15}$-arylalkyl or $C_4$-$C_{14}$-aryl, or $NR^{14}R^{15}$ together is a cyclic amino radical having 4 to 20 carbon atoms, or $R^3$ and $R^4$ together are $-OR^{16}-O-$ where $R^{16}$ is a radical selected from the group of $C_2$-$C_4$-alkylene, 1,2-phenylene, 1,3-phenylene, 1,2-cyclohexylene, 1,1'-ferrocenylene, 1,2-ferrocenylene, 2,2'-(1,1'-binaphthylene), 2,2'-(1,1')-biphenylene and 1,1'-(diphenyl-2,2'-methylene)diyl, and the radicals mentioned may optionally be mono- or polysubstituted by radicals selected from the group of fluorine, chlorine, $C_1$-$C_8$-alkoxy and $C_1$-$C_8$-alkyl and
- $R^5$ is hydrogen, $C_1$-$C_{20}$-alkyl, $C_4$-$C_{24}$-aryl, $C_5$-$C_{25}$-arylalkyl, $C_1$-$C_{20}$-haloalkyl or a radical of the formula (IIb)

$$A\text{-}B\text{-}D \qquad \text{(IIb)}$$

where

A is absent or is $C_1$-$C_{12}$-alkylene

B is a functionality which is selected from the group of

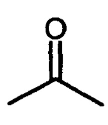 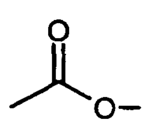 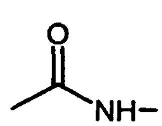 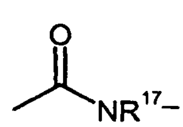

where

$R^{17}$ may be $C_1$-$C_{20}$-alkyl, $C_4$-$C_{24}$-aryl, $C_5$-$C_{25}$-arylalkyl

and

D is $C_1$-$C_8$-alkyl, $C_4$-$C_{24}$-aryl or $C_5$-$C_{25}$-arylalkyl or

B and D, in the case that A is not absent, may together be cyano or $[(C_1$-$C_8$-alkylene)-O$]_n$-($C_1$-$C_8$-alkyl) where n is an integer between 1 and 8 or

$R^{17}$ and D together are a cyclic amino radical having 4 to 12 carbon atoms.

**2.** Compounds according to Claim 1, **characterized in that** *1, *2, *3 and *4 together define the following stereoisomers of the central substituted furan ring:
(1R,2R,3R,4R), (1R,2R,3R,4S), (1R,2S,3S,4S), (1R,2S,3S,4R), (1R,2R,3S,4R), (1S,2S,3R,4S), (1S,2S,3S,4S), (1S,2S,3S,4R), (1S,2R,3R,4R), (1S,2R,3R,4S), (1S,2S,3R,4S), (1R,2R,3S,4R).

**3.** Compounds according to at least one of Claims 1 and 2, **characterized in that** $R^1$ and $R^2$ are each independently hydrogen, tert-butoxy, trityloxy, tert-butyldimethylsilyloxy, tert-butyldiphenylsilyloxy, trimethylsilyloxy, triethylsilyloxy, triisopropylsilyloxy, neopentoxy or 1-adamantoxy.

**4.** Compounds according to at least one of Claims 1 to 3, **characterized in that** $R^1$ and $R^2$ are identical.

**5.** Compounds according to at least one of Claims 1 to 4, **characterized in that** $R^3$ and $R^4$ are each independently $R^{12}$, $OR^{13}$ or $NR^{14}R^{15}$ where $R^{12}$, $R^{13}$, $R^{14}$ and $R^{15}$ are each independently $C_1$-$C_{12}$-alkyl or $C_4$-$C_{14}$-aryl, or $NR^{14}R^{15}$ together is a cyclic amino radical having 4 to 12 carbon atoms, for example pyrrolidinyl or piperidinyl or $R^3$ and $R^4$ together are -O-$R^{16}$-O- where $R^{16}$ is ethylene, 1,2-phenylene, 1,3-phenylene, 1,2-cyclohexylene, 1,1'-ferrocenylene, di- or tetra-$C_1$-$C_8$-alkyl-substituted 1,1'-(diphenyl-2,2'-methylene)diyl, 1,2-ferrocenylene, 2,2'-(1,1'-binaphthylene) or 2,2'-(1,1')-biphenylene, and 2,2'-(1,1'-binaphthylene) or 2,2'-(1,1')-biphenylene is substituted at least in the 6,6'-position by radicals which are selected from the group of $C_1$-$C_8$-alkoxy and $C_1$-$C_8$-alkyl, and may also be substituted in the 5,5'-, 4,4'-, 3,3'- or 2,2'-position by radicals which are selected from the group of fluorine, chlorine, $C_1$-$C_8$-alkoxy and $C_1$-$C_8$-alkyl.

**6.** Compounds according to at least one of Claims 1 to 5, **characterized in that** $R^5$ is hydrogen, $C_1$-$C_4$-alkyl, -CO($C_1$-$C_4$-alkyl), benzyl-CO-phenyl or phenyl, and benzyl or phenyl may optionally be further substituted by one, two or three substituents selected from the group of $C_1$-$C_4$-alkyl, $C_1$-$C_4$-alkoxy or $C_1$-$C_4$-haloalkyl.

**7.** Compounds according to at least one of Claims 1 to 6, **characterized in that** they obey the formulae (Ia) to (Id)

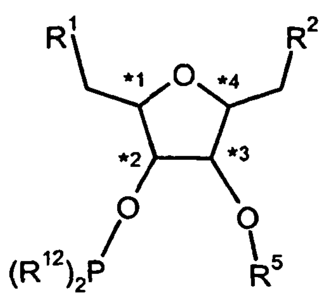

(Ia)

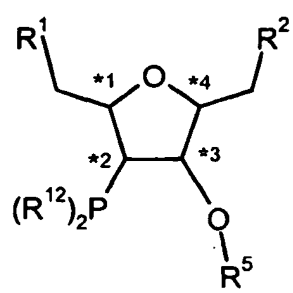

(Ib)

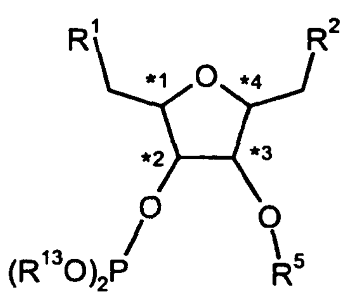

(Ic)

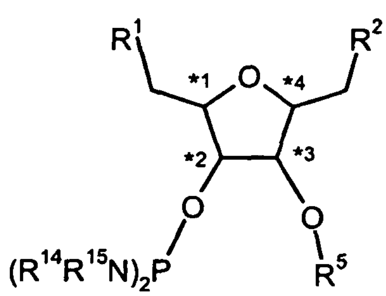

(Id)

where *1,*2,*3,*4, $R^1$, $R^2$, $R^5$, $R^{12}$, $R^{13}$, $R^{14}$ and $R^{15}$ are as defined under formula (I).

**8.** 2-*O*-(Di(2,4-dimethylphenyl)phosphino)-1,6-di-*O*-(*tert*-butyldiphenylsilyl)-2,5-anhydro-D-mannitol.

**9.** Process for preparing compounds of the formula (Ib)

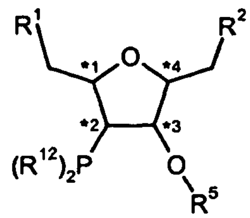

(Ib)

where

$R^1$, $R^2$, $R^5$, $R^6$ and $R^{12}$ are as defined under formula (I), **characterized in that** compounds of the formula (XV)

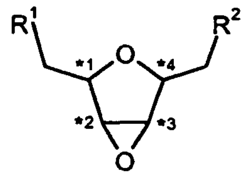

(XV)

where

$R^1$ and $R^2$ are as defined under formula (I), in the presence of compounds of the formula (XVI),

$$(R^{12})_2PMet^2 \qquad (XVI)$$

where

$Met^2$ is lithium, sodium or potassium and

$R^{12}$ has the definition and areas of preference specified under (I),

are converted to compounds of the formula (XVII)

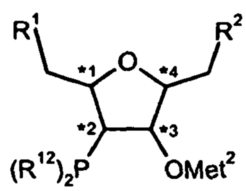

(XVII)

where
$R^1$, $R^2$, $Met^2$ and $R^{12}$ are as defined above,
and the compounds of the formula (XVII) are reacted with compounds of the formula (XIII),

$$R^5Z \qquad (XIII)$$

where

$R^5$ has the same definitions and areas of preference as specified under formula (I) and

Z is chlorine, bromine, iodine or $R^{19}SO_3$ where $R^{19}$ is $C_1$-$C_{12}$-alkyl, $C_1$-$C_{12}$-haloalkyl, $C_5$-$C_{25}$-arylalkyl or $C_4$-$C_{24}$-aryl, and, in the case that $R^5$ is to be bonded via a carbonyl group, may also be $R^5O$-.

**10.** Transition metal complexes containing compounds according to one or more of Claims 1 to 8.

**11.** Transition metal complexes according to Claim 10, **characterized in that** the transition metal is selected from the group of ruthenium, osmium, cobalt, rhodium, iridium, nickel, palladium, platinum and copper.

**12.** Transition metal complexes according to at least one of Claims 10 and 11, **characterized in that** the molar ratio of transition metal to compounds according to at least one of Claims 1 to 8 is 1:2, 1:3 or 1:4.

**13.** Transition metal complexes according to at least one of Claims 10 and 11, **characterized in that** they obey the formula (XIX)

$$[(I)_4M] \qquad (XIX)$$

where

(I) is a compound of the formula (I) as defined in claim 1 and

M is rhodium or iridium.

**14.** Transition metal complexes according to at least one of Claims 10 and 11, **characterized in that** they are obtained by reacting transition metal compounds and compounds according to at least one of Claims 1 to 8.

**15.** Transition metal complexes according to Claim 14, **characterized in that** the transition metal compounds used are:

transition metal compounds of the formula (XXa)

$$M(An^1)_q \qquad (XXa)$$

where

M is rhodium, iridium, ruthenium, nickel, palladium, platinum or copper and

$An^1$ is chloride, bromide, acetate, nitrate, methanesulphonate, trifluoromethanesulphonate or acetylacetonate and

q is 3 for rhodium, iridium and ruthenium, is 2 for nickel, palladium and platinum, and is 1 for copper,

or transition metal compounds of the formula (XXb)

$$M(An^2)_qL^1{}_2 \qquad (XXb)$$

where

M is ruthenium, iridium, ruthenium, nickel, palladium, platinum or copper and

$An^2$ is chloride, bromide, acetate, methanesulphonate or trifluoromethanesulphonate, tetrafluoroborate or hexafluorophosphate, perchlorate, hexafluoroantimonate, tetra(bis-3,5-trifluoromethylphenyl)borate or tetraphenylborate and

q is 1 for rhodium and iridium, is 2 for ruthenium, nickel, palladium and platinum, and is 1 for copper,

$L^1$ is in each case $C_2$-$C_{12}$-alkene, for example ethylene or cyclooctene, or a nitrile, for example acetonitrile, benzonitrile or benzyl nitrile, or

$L^1_2$ together is a ($C_4$-$C_{12}$)-diene, for example bicyclo[2.1.1]hepta-2,5-diene (norbornadiene) or 1,5-cyclooctadiene,

or transition metal compounds of the formula (XXc)

$$[ML^2An^1{}_2]_2 \quad (XXc)$$

where

M is ruthenium and

$L^2$ is an aryl radical, for example cymene, mesityl, phenyl or cyclooctadiene, norbornadiene or methylallyl,

or transition metal compounds of the formula (XXd)

$$Met^3{}_q[M(An^3)_4] \quad (XXd)$$

where

M is palladium, nickel, iridium or rhodium and

$An^3$ is chloride or bromide and

$Met^3$ is lithium, sodium, potassium, ammonium or an organic ammonium ion and

q is 3 for rhodium and iridium, and is 2 for nickel, palladium and platinum,

or transition metal compounds of the formula (XXe)

$$[M(L^3)_2]An^4 \quad (XXe)$$

where

M is iridium or rhodium and

$L^3$ is ($C_4$-$C_{12}$)-diene, for example bicyclo[2.1.1]hepta-2,5-diene (norbomadiene) or 1,5-cyclooctadiene and

$An^4$ is an uncoordinating or weakly coordinating anion, for example methanesulphonate, trifluoromethanesulphonate, tetrafluoroborate, hexafluorophosphate, perchlorate, hexafluoroantimonate, tetra(bis-3,5-trifluoromethylphenyl)borate or tetraphenylborate,

or Ni(1,5-cyclooctadiene)$_2$, $Pd_2$(dibenzylideneacetone)$_3$, $Pd[PPh_3]_4$, cyclopentadienyl$_2$Ru, Rh(acac)$(CO)_2$, Ir (pyridine)2(1,5-cyclooctadiene), Cu(phenyl)Br, Cu(phenyl)Cl, Cu(phenyl)I, $Cu(PPh_3)_2Br$, $[Cu(CH_3CN)_4]BF_4$ and $[Cu(CH_3CN)_4]PF_6$ or multinuclear bridged complexes, for example [Rh(1,5-cyclooctadiene)Cl]$_2$, [Rh (1,5-cyclooctadiene)Br]$_2$, [Rh(ethene)$_2$Cl]$_2$, and [Rh(cyclooctene)$_2$Cl]$_2$.

**16.** Transition metal complexes according to at least one of Claims 14 and 15, **characterized in that** the amount of the metal in the transition metal compounds used is from 5 to 100 mol%, based on the compound according to at

least one of Claims 1 to 8 used.

**17.** Catalysts containing transition metal complexes according to at least one of Claims 10 to 16.

**18.** Use of transition metal complexes according to one or more of Claims 10 to 16 or catalysts according to Claim 17 for preparing stereoisomerically enriched compounds.

**19.** Use according to Claim 18, **characterized in that** the stereoisomerically enriched compounds are obtained by asymmetric 1,4-additions, asymmetric hydroformylations, asymmetric hydrocyanations, asymmetric Heck reactions and asymmetric hydrogenations.

**20.** Use according to one or both of Claims 18 and 19, **characterized in that** the stereoisomerically enriched compounds are used for preparing active ingredients in pharmaceuticals and agrochemicals, or intermediates of both of these classes.

**21.** Process for preparing stereoisomerically enriched compounds by catalytic hydrogenations of olefins, enamines, enamides, imines or ketones, 1,4-additions, hydroformylations, hydrocyanations or Heck reactions, **characterized in that** the catalysts used are those which contain transition metal complexes according to one or more of Claims 10 to 16.

**22.** Process according to Claim 21, **characterized in that** the amount of the transition metal complexes used is 0.001 to 5 mol%, based on the substrate used.

**23.** Process according to one or more of Claims 21 and 22, **characterized in that** the stereoisomerically enriched compounds are obtained by catalytic hydrogenation of olefins, enamides or imines.

**24.** Process according to one or more of Claims 21 to 23, **characterized in that** the working temperature is -20°C to 200°C.

**25.** Process according to one or more of Claims 21 to 24, **characterized in that** the hydrogen pressure is 0.1 to 200 bar.

## Revendications

**1.** Composés de formule (I)

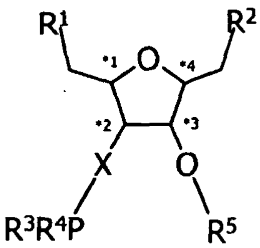

(I)

dans laquelle

- *1, *2, *3 et *4 signalent chacun, indépendamment les uns des autres, un atome de carbone stéréogène qui est en configuration R ou S,
- X manque ou représente l'oxygène et
- $R^1$ et $R^2$ représentent chacun, indépendamment l'un de l'autre, l'hydrogène, un groupe alkyle en $C_1$-$C_{20}$, fluoroalkyle en $C_1$-$C_{20}$, alcényle en $C_2$-$C_{20}$, aryle en $C_4$-$C_{24}$, arylalkyle en $C_5$-$C_{25}$, arylalcényle en $C_6$-$C_{26}$ ou $NR^6R^7$, $OR^7$, -(alkyle en $C_1$-$C_8$)-$OR^7$, -(alkyle en $C_1$-$C_8$)-$NR^6R^7$ ou -$O_2CR^7$,
  $R^6$ et $R^7$ représentant chacun, indépendamment l'un de l'autre, un groupe alkyle en $C_1$-$C_8$, arylalkyle en $C_5$-$C_{15}$ ou aryle en $C_4$-$C_{14}$,
  ou bien formant ensemble un groupe cyclique aminé contenant au total 4 à 20 atomes de carbone,
  ou bien $R^1$ et $R^2$ représentent chacun, indépendamment l'un de l'autre, un groupe de formule (IIa)

$$-R^8\text{-}SiR^9R^{10}R^{11} \qquad \text{(IIa)}$$

dans laquelle
$R^8$ manque ou représente l'oxygène ou un groupe méthylène et
$R^9$, $R^{10}$ et $R^{11}$ représentent chacun, indépendamment les uns des autres, un groupe alkyle en $C_1$-$C_{12}$, arylalkyle en $C_5$-$C_{15}$ ou aryle en $C_6$-$C_{14}$ et

- $R^3$ et $R^4$ représentent chacun, indépendamment l'un de l'autre, $R^{12}$, $OR^{13}$ ou $NR^{14}R^{15}$, $R^{12}$, $R^{13}$, $R^{14}$ et $R^{15}$ représentant chacun, indépendamment les uns des autres, un groupe alkyle en $C_1$-$C_{12}$, arylalkyle en $C_5$-$C_{15}$ ou aryle en $C_4$-$C_{14}$ ou bien $NR^{14}R^{15}$ représentent ensemble un groupe cyclique aminé contenant 4 à 20 atomes de carbone, ou bien $R^3$ et $R^4$ représentent ensemble un groupe $-OR^{16}-O-$, $R^{16}$ représentant un groupe choisi parmi les groupes alkylène en $C_2$-$C_4$, 1,2-phénylène, 1,3-phénylène, 1,2-cyclohexylène, 1,1'-ferrocénylène, 1,2-ferrocénylène, 2,2'-(1,1'-binaphtylène), 2,2'-(1,1')-biphénylène et 1,1'-(diphényl-2,2'-méthylène)-diyle, ces groupes pouvant le cas échéant porter un ou plusieurs substituants choisis parmi le fluor, le chlore, les groupes alcoxy en $C_1$-$C_8$ et alkyle en $C_1$-$C_8$, et
- $R^5$ représente l'hydrogène, un groupe alkyle en $C_1$-$C_{20}$, aryle en $C_4$-$C_{24}$, arylalkyle en $C_5$-$C_{25}$, halogénoalkyle en $C_1$-$C_{20}$ ou un groupe de formule (IIb)

$$\text{A-B-D} \qquad \text{(IIb)}$$

dans laquelle

A manque ou représente un groupe alkylène en $C_1$-$C_{12}$,
B représente une fonctionnalité choisie parmi les suivantes :

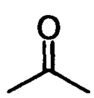 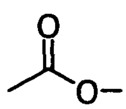 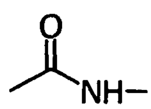 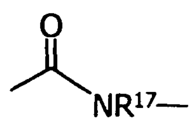

dans lesquelles

$R^{17}$ représente un groupe alkyle en $C_1$-$C_{20}$, aryle en $C_4$-$C_{24}$, arylalkyle en $C_5$-$C_{25}$ et
D représente un groupe alkyle en $C_1$-$C_8$, aryle en $C_4$-$C_{24}$ ou arylalkyle en $C_5$-$C_{25}$, ou bien
B et D, dans les cas où A ne manque pas, représentent ensemble un groupe cyano ou [(alkylène en $C_1$-$C_8$)-O]$_n$-alkyle en $C_1$-$C_8$, n étant un nombre entier qui va de 1 à 8, ou bien
$R^{17}$ et D représentent ensemble un groupe cyclique aminé contenant 4 à 12 atomes de carbone.

2. Composés selon la revendication 1, **caractérisés en ce que** *1, *2, *3 et *4 signalent ensemble les stéréo-isomères suivants du cycle furanne substitué central :
(1R,2R,3R,4R), (1R,2R,3R,4S), (1R,2S,3S,4S), (1R,2S,3S,4R), (1R,2R,3S,4R), (1S,2S,3R,4R), (1S,2S,3S,4S), (1S,2S,3S,4R), (1S,2R,3R,4R), (1S,2R,3R,4S), (1S,2S,3R,4S), (1R,2R,3S,4R),

3. Composés selon au moins une des revendications 1 et 2, **caractérisés en ce que** $R^1$ et $R^2$ représentent chacun, indépendamment l'un de l'autre, l'hydrogène, un groupe tert.-butoxy, trityloxy, tert-butyldiméthylsilyloxy, tert-butyldiphénylsilyloxy, triméthysilyloxy, triéthylsilyloxy, triisopropylsilyloxy, néo-pentoxy ou 1-adamantoxy.

4. Composés selon au moins une des revendications 1 à 3, **caractérisés en ce que** $R^1$ et $R^2$ sont identiques.

5. Composés selon au moins une des revendications 1 à 4, **caractérisés en ce que** $R^3$ et $R^4$ représentent chacun, indépendamment l'un de l'autre, $R^{12}$, $OR^{13}$ ou $NR^{14}R^{15}$, $R^{12}$, $R^{13}$, $R^{14}$ et $R^{15}$ représentant chacun, indépendamment les uns des autres, un groupe alkyle en $C_1$-$C_{12}$ ou aryle en $C_4$-$C_{14}$, ou bien $NR^{14}R^{15}$ représentant ensemble un groupe cyclique aminé contenant 4 à 12 atomes de carbone, par exemple un groupe pyrrolidinyle ou pipéridinyle, ou bien $R^3$ et $R^4$ représentent ensemble un groupe $-OR^{16}-O-$, $R^{16}$ représentant un groupe éthylène, 1,2-phénylène,

1,3-phénylène, 1,2-cyclohexylène, 1,1'-ferrocénylène, un groupe 1,1'-(diphényl-2,2'-méthylène)-diyle, portant deux ou quatre substituant alkyle en $C_1$-$C_8$, un groupe 1,2-ferrocénylène, 2,2'-(1,1')-binaphtylène) ou 2,2'-(1,1')-biphénylène), le groupe 2,2'-(1,1'-binaphtylène) ou le groupe 2,2'-(1,1')-biphénylène) étant substitué au moins en position 6,6' par des groupes choisis parmi les groupes alcoxy en $C_1$-$C_8$ et alkyle en $C_1$-$C_8$, et pouvant en outre porter en position 5,5' ; 4,4' ; 3,3' ou 2,2' des substituants choisis parmi le fluor, le chlore, les groupes alcoxy en $C_1$-$C_8$ et alkyle en $C_1$-$C_8$.

6. Composés selon au moins une des revendications 1 à 5, **caractérisés en ce que** $R^5$ représente l'hydrogène, un groupe alkyle en $C_1$-$C_4$, -CO(alkyle en $C_1$-$C_4$), benzylCOphényle ou phényle, les parties benzyle et phényle pouvant le cas échéant porter encore un, deux ou trois substituants choisis parmi les groupes alkyle en $C_1$-$C_4$, alcoxy en $C_1$-$C_4$ et halogénoalkyle en $C_1$-$C_4$.

7. Composés selon au moins une des revendications 1 à 6, **caractérisés en ce qu'**ils répondent aux formules (Ia) à (Id)

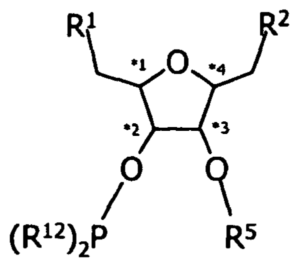

(Ia)

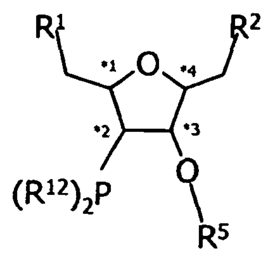

(Ib)

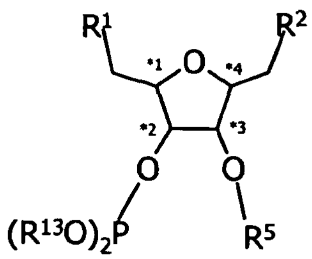

(Ic)

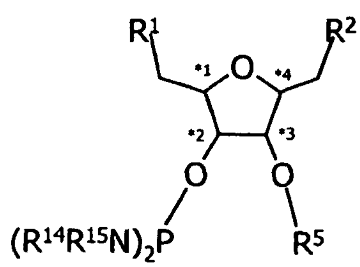

(Id)

dans lesquelles *1, *2, *3, *4, $R^1$, $R^2$, $R^5$, $R^{12}$, $R^{13}$, $R^{14}$ et $R^{15}$ ont les significations indiquées en référence à la formule (I).

8. Le 2-*O*-[di(2,4-diméthylphényl)phosphino]-1,6-di-*O*-(*tert*-butyldiphénylsilyl)-2,5-anhydro-D-mannitol.

9. Procédé pour la préparation des composés de formule (Ib)

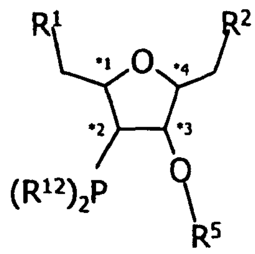

(Ib)

dans laquelle
$R^1$, $R^2$, $R^5$, $R^6$ et $R^{12}$ ont les significations indiquées dans la revendication 1 en référence à la formule (I), **caractérisé en ce que** l'on fait réagir des composés de formule (XV)

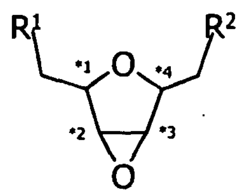

(XV)

dans laquelle
$R^1$ et $R^2$ ont les significations indiquées en référence à la formule (I), en présence de composés de formule (XVI)

$$(R^{12})_2PMet_2 \qquad (XVI)$$

dans laquelle

Met$^2$ représente le lithium, le sodium ou le potassium et
$R^{12}$ a les significations, y compris les significations préférées, indiquées en référence à la formule (I),

la réaction donnant des composés de formule (XVII)

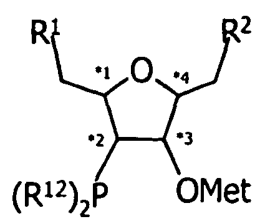

(XVII)

dans laquelle
$R^1$, $R^2$, Met$^2$ et $R^{12}$ ont les significations indiquées ci-dessus, après quoi on fait réagir les composés de formule (XVII) avec des composés de formule (XIII)

$$R^5Z \qquad (XIII)$$

dans laquelle

$R^5$ a les significations, y compris les significations préférées, indiquées en référence à la formule (I) et
Z représente le chlore, le brome, l'iode ou $R^{19}SO_3$, $R^{19}$ représentant un groupe alkyle en $C_1$-$C_{12}$, halogénoalkyle en $C_1$-$C_{12}$, arylalkyle en $C_5$-$C_{25}$ ou aryle en $C_4$-$C_{24}$, ou bien encore, lorsque $R^5$ est relié par l'intermédiaire d'un groupe carbonyle, un groupe $R^5O$-.

**10.** Complexes de métaux de transition contenant des composés selon une ou plusieurs des revendications 1 à 8.

**11.** Complexes de métaux de transition selon la revendication 10, **caractérisés en ce que** le métal de transition est choisi dans le groupe consistant en le ruthénium, l'osmium, le cobalt, le rhodium, l'iridium, le nickel, le palladium, le platine et le cuivre.

**12.** Complexes de métaux de transition selon au moins une des revendications 10 à 11, **caractérisés en ce que** le rapport molaire entre le métal de transition et les composés selon au moins une des revendications 1 à 8 est de 1:2, 1:3 ou 1:4.

**13.** Complexes de métaux de transition selon au moins une des revendications 10 à 11, **caractérisés en ce qu'**ils répondent à la formule (XIX)

$$[(I)_4M] \quad \text{(XIX)}$$

dans laquelle

(I) représente des composés de formule (I), avec les significations indiquées dans la revendication 1 et
M représente le rhodium ou l'iridium.

**14.** Complexes de métaux de transition selon au moins une des revendications 10 à 11, **caractérisés en ce qu'**ils ont été obtenus par réaction entre des composés de métaux de transition et des composés selon au moins une des revendications 1 à 8.

**15.** Complexes de métaux de transition selon la revendication 14, **caractérisés en ce que** l'on a utilisé les composés de métaux de transition suivants :

les composés de métaux de transition de formule (XXa)

$$M(An^1)_q \quad \text{(XXa)}$$

dans laquelle

M représente le rhodium, l'iridium, le ruthénium, le nickel, le palladium, le platine ou le cuivre et
$An^1$ représente un chlorure, un bromure, un acétate, un nitrate, un méthanesulfonate, un trifluorométhanesulfonate ou un acétylacétonate et
q est égal à 3 pour le rhodium, l'iridium et le ruthénium, à 2 pour le nickel, le palladium et le platine et à 1 pour le cuivre,

ou les composés de métaux de transition de formule (XXb)

$$(M(An^2)_qL^1{}_2 \quad \text{(XXb)}$$

dans laquelle

M représente le ruthénium, l'iridium, le nickel, le palladium, le platine ou le cuivre et
$An^2$ représente un chlorure, un bromure, un acétate, un méthanesulfonate ou un trifluorométhanesulfonate, un tétrafluoroborate ou un hexafluorophosphate, un perchlorate, un hexafluoroantimoniate, un tétra(bis-3,5-trifluorométhylphényl)-borate ou un tétraphénylborate et

q est égal à 1 pour le rhodium et l'iridium, à 2 pour le ruthénium, le nickel, le palladium et le platine et à 1 pour le cuivre,

chacun des symboles $L^1$ représente un alcène en $C_2$-$C_{12}$, par exemple l'éthylène ou le cyclooctène, ou un nitrile, par exemple l'acétonitrile, le benzonitrile ou le benzylnitrile, ou bien

$L^1_2$ représentent ensemble un diène en $C_4$-$C_{12}$, par exemple le bicyclo[2.1.1]hepta-2,5-diène (norbornadiène) ou le 1,5-cyclooctadiène,

ou les composés de métaux de transition de formule (XXc)

$$[ML^2An^1{}_2]_2 \qquad (XXc)$$

dans laquelle

M représente le ruthénium et
$L^2$ représente un groupe aryle, par exemple cyményle, mésityle, phényle ou un groupe cyclooctadiène, norbornadiène ou méthylallyle,

ou les composés de métaux de transition de formule (XXd)

$$Met^3{}_q[M(An^3)_4] \qquad (XXd)$$

dans laquelle

M représente le palladium, le nickel, l'iridium ou le rhodium et
$An^3$ représente un chlorure ou un bromure,
$Met^3$ représente le lithium, le sodium, le potassium, l'ammonium ou un ammonium organique et
q est égal à 3 pour le rhodium et l'iridium, à 2 pour le nickel, le palladium et le platine,

ou les composés de métaux de transition de formule (XXe)

$$[M(L^3)_2]An^4 \qquad (XXe)$$

dans laquelle

M représente l'iridium ou le rhodium et
$L^3$ représente un diène en $C_4$-$C_{12}$, par exemple le bicyclo[2.1.1]hepta-2,5-diène (norbornadiène) ou le 1,5-cyclooctadiène et
$An^4$ représente un anion non coordonné ou faiblement coordonné, par exemple un anion méthanesulfonate, trifluorométhanesulfonate, tétrafluoroborate, hexafluorophosphate, perchlorate, hexafluoroantimoniate, tétra(bis-3,5-trifluorométhylphényl)borate ou tétraphénylborate,

ou bien Ni(1,5-cyclooctadiène)$_2$, $Pd_2$(dibenzylidénacétone)$_3$, Pd[$PPh_3$]$_4$, (cyclopentadiényl)$_2$Ru, Rh(acac)$(CO)_2$, Ir(pyridine)2(1,5-cyclooctadiène), Cu(phényl)Br, Cu(phényl)Cl, Cu(phényl)I, $Cu(PPh_3)_2Br$, $[Cu(CH_3CN)_4]BF_4$ et $[Cu(CH_3CN)_4]PF_6$ ou des complexes polycycliques pontés tels que [Rh(1,5-cydooctadiène)Cl]$_2$, [Rh(1,5-cyclooctadiène)Br]$_2$, [Rh(éthène)$_2$Cl]$_2$, et [Rh(cyclooctène)$_2$Cl]$_2$.

**16.** Complexes de métaux de transition selon au moins une des revendications 14 à 15 **caractérisés en ce que**, dans les composés de métaux de transition mis en oeuvre, la quantité du métal représente de 5 à 100 mol % du composé selon au moins une des revendications 1 à 8 mis en oeuvre.

**17.** Catalyseurs contenant des complexes de métaux de transition selon au moins une des revendications 10 à 16.

**18.** Utilisation des complexes de métaux de transition selon une ou plusieurs des revendications 10 à 16 ou de catalyseurs selon la revendication 17 pour la préparation de composés enrichis en stéréo-isomères.

**19.** Utilisation selon la revendication 18 **caractérisée en ce que** les composés enrichis en stéréo-isomères sont obtenus par des 1,4-additions asymétriques, des hydroformylations asymétriques, des hydrocyanations asymétriques, des réactions de Heck asymétriques et des hydrogénations asymétriques.

**20.** Utilisation selon une ou plusieurs des revendications 18 et 19, **caractérisée en ce que** les composés enrichis en stéréo-isomères sont utilisés pour la préparation de substances actives médicamenteuses ou agrochimiques ou de produits intermédiaires des mêmes classes.

**21.** Procédé pour la préparation de composés enrichis en stéréo-isomères par hydrogénation catalytiques d'oléfines, d'énamines, d'énamides, d'imines ou de cétones, des 1,4-additions, des hydroformylations, des hydrocyanations ou des réactions de Heck, **caractérisé en ce que** l'on utilise des catalyseurs contenant des complexes de métaux de transition selon une ou plusieurs des revendications 10 à 16.

**22.** Procédé selon la revendication 21 **caractérisé en ce que** les complexes de métaux de transition sont mis en oeuvre en proportions de 0,001 à 5 mol % par rapport au substrat mis en oeuvre.

**23.** Procédé selon une ou plusieurs des revendications 21 et 22, **caractérisé en ce que** les composés enrichis en stéréo-isomères sont obtenus par hydrogénation catalytique d'oléfines, d'énamides ou d'imines.

**24.** Procédé selon une ou plusieurs des revendications 21 à 23, **caractérisé en ce que** l'on opère à des températures allant de -20 à 200°C.

**25.** Procédé selon une ou plusieurs des revendications 21 à 24, **caractérisé en ce que** l'on opère sous une pression d'hydrogène de 0,1 à 200 bar.